# DEMANDE DE BREVET EUROPEEN

(11) **EP 1 568 686 A1**
(43) Date de publication de la demande: **31.08.2005**
(21) Numéro de dépôt: 05290424.0
(22) Date de dépôt: 24.02.2005
(51) Int. Cl.: C07C 233/36, C07C 237/06, C07C 211/53, C07D 295/00, C07D 207/00, C07D 211/00

(54) **Para-phénylénediamine secondaire n-alkylaminée ortho- et/ou méta-substituée, composition de teinture des fibres kératiniques contenant une telle paraphénylènediamine, procédés mettant en oeuvre cette composition et utilisations**

(30) Priorité: 27.02.2004 FR 0402021
(71) Demandeur: L'OREAL, 75008 Paris (FR)
(72) Inventeur: Sabelle, Stéphane, 75005 Paris (FR)
(74) Mandataire: Dossmann, Gérard

(57) **Abrégé**

La présente demande concerne de nouvelles para-phénylènediamines secondaires N-alkylaminées ortho et/ou méta-substituées, une composition pour la teinture des fibres kératiniques, et en particulier les fibres kératiniques humaines telles que les cheveux, contenant, dans un milieu approprié pour la teinture, au moins une para-phénylènediamine secondaire N-alkylaminée ortho et/ou méta-substituée, un procédé de teinture des fibres kératiniques qui consiste à appliquer cette composition ainsi que les utilisations de cette composition en particulier sous la forme d'un "kit" de teinture.

## Description

La présente invention a pour objet une nouvelle famille de para-phénylènediamines secondaires N-alkylaminées ortho substituées et/ou méta substituées et leur utilisation pour la teinture des fibres kératiniques, en particulier des fibres kératiniques humaines telles que les cheveux.

Il est connu de teindre les fibres kératiniques et en particulier les cheveux humains avec des compositions tinctoriales contenant des précurseurs de colorants d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance à des composés colorés par un processus de condensation oxydative.

On sait également que l'on peut faire varier les nuances obtenues avec ces bases d'oxydation en les associant à des coupleurs ou modificateurs de coloration, ces derniers étant choisis notamment parmi les méta-diaminobenzènes aromatiques, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

De manière surprenante et avantageuse, la Demanderesse vient de découvrir qu'il est possible d'obtenir de nouvelles compositions pour la teinture des fibres kératiniques, en particulier des fibres kératiniques humaines telles que les cheveux, capables de conduire à des colorations aux nuances variées, puissantes, esthétiques, peu sélectives et résistant bien aux diverses agressions que peuvent subir les fibres, en utilisant au moins une para-phénylènediamine secondaire N-alkylaminée ortho substituée et/ou méta substituée.

En outre, ces compositions présentent un bon profil toxicologique.

Un premier objet de l'invention concerne une famille de para-phénylènediamines secondaires N-alkylaminées ortho substituées et/ou méta substituées, leurs procédés de synthèse et leurs utilisations en particulier pour la teinture des fibres kératiniques, en particulier les fibres kératiniques humaines telles que les cheveux.

L'invention a aussi pour objet une composition contenant au moins une para-phénylènediamine secondaire N-alkylaminée ortho substituée et/ou méta substituée, les procédés de teinture mettant en oeuvre cette composition, les utilisations de la composition selon la présente invention pour la teinture des fibres kératiniques, en particulier les fibres kératiniques humaines telles que les cheveux et en particulier, les dispositifs à plusieurs compartiments ou "kits" de teinture.

La composition de la présente invention permet en particulier d'obtenir une coloration des fibres kératiniques très puissante, peu sélective et tenace, en particulier à la lumière, tout en évitant la dégradation de ces fibres.

D'autres caractéristiques, aspects, objets et avantages de la présente invention apparaîtront encore plus clairement à la lecture de la description et des exemples qui suivent.

Dans le cadre de la présente invention, on entend par alkyle, un radical linéaire ou ramifié en C₁-C₁₀, par exemple les radicaux linéaires ou ramifiés suivants : méthyle, éthyle, propyle, butyle, pentyle, hexyle, octyle...

Les nouvelles para-phénylènediamines secondaires N-alkylaminées ortho substituées et/ou méta substituées selon la présente invention sont des composés de formule générale (I) : 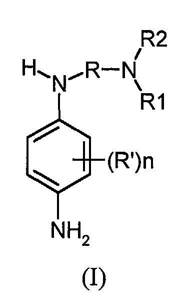 dans laquelle :
les radicaux R₁ et R₂ représentent, indépendamment l'un de l'autre, un atome d'hydrogène, un groupement alkyle, ou les radicaux R₁ et R₂ forment, ensemble et avec l'azote qu'ils substituent, un cycle saturé à 5 ou 6 chaînons éventuellement interrompu par un ou plusieurs atomes d'azote et/ou par un ou plusieurs groupements carbonyle, et éventuellement substitué par un ou plusieurs groupements alkyles,
le radical R représente un radical alkylène en C₂-C₁₀, linéaire ou ramifié, éventuellement interrompu par un ou plusieurs atomes choisis parmi l'azote ou l'oxygène, et éventuellement substitué par un ou plusieurs groupements choisis parmi les groupements amino, monoalkylamino, dialkylamino, alkylcarbonyle, amido, alkoxycarbonyle, monoalkylaminocarbonyle, dialkylaminocarbonyle,
le radical R' représente un radical choisi parmi les radicaux alkyle, alcoxy, alcoxyalkyle, monohydroxyalkyle , polyhydroxyalkyle et l'atome de chlore,
n est un entier égal à 1,
sous réserve que le composé de formule (I) ne soit pas un des composés suivants : N-1-(2-Diethylamino-ethyl)-2-methoxy-benzene-1,4-diamine ; N-1-(3-Diethylamino-propyl)-3-methyl-benzene-1,4-diamine, et le N-1-(3-pyrrolidino-propyl)-3-methyl-benzene-1,4-diamine.

De manière préférée, R₁ représente un atome d'hydrogène ou un groupement alkyle, R₂ étant tel que défini ci-dessus ; ou les radicaux R₁ et R₂ forment ensemble avec l'azote qu'ils substituent un cycle saturé à 5 ou 6 chaînons éventuellement interrompu par un ou plusieurs atomes d'azote, et éventuellement substitué par un ou plusieurs groupements alkyles.

De manière encore préférée, le radical R₁ représente un atome d'hydrogène, et le radical R₂ représente un groupement choisi parmi l'hydrogène ou un groupement alkyle; ou R₁ et R₂ représentent tous les deux des groupements alkyle, de manière encore préférée les deux groupements alkyles sont identiques ; ou encore R₁ et R₂ forment ensemble avec l'azote qu'ils substituent un cycle saturé à 5 ou 6 chaînons éventuellement interrompu par un ou plusieurs atomes d'azote et/ou un ou plusieurs groupements carbonyles, ou éventuellement substitué par un groupement alkyle en C₁-C₃, de préférence R représente un radical alkylène linéaire ou ramifié en C₂-C₈, éventuellement interrompu par un ou deux atomes d'azote.

De manière préférée, le radical R représente un radical alkylène linéaire ou ramifié en C₂-C₈, éventuellement interrompu par un ou deux atomes d'azote.

De manière préférée, le radical R' est un groupement choisi parmi les alkyle en C₁-C₃, alcoxy en C₁-C₃, alcoxy en (C₁-C₃)-alkyle en (C₁-C₃) et l'atome de chlore.

Les composés de formule (I) peuvent se présenter sous forme libre ou sous forme de sels, tel que les sels d'addition avec un acide de préférence choisis parmi les chlorhydrates, les bromhydrates, les sulfates, les citrates, les succinates, les tartrates, les lactates, les tosylates, les benzènesulfonates, les phosphates et les acétates .

Quelques composés de formule (I) préférés sont présentés dans le tableau qui suit :

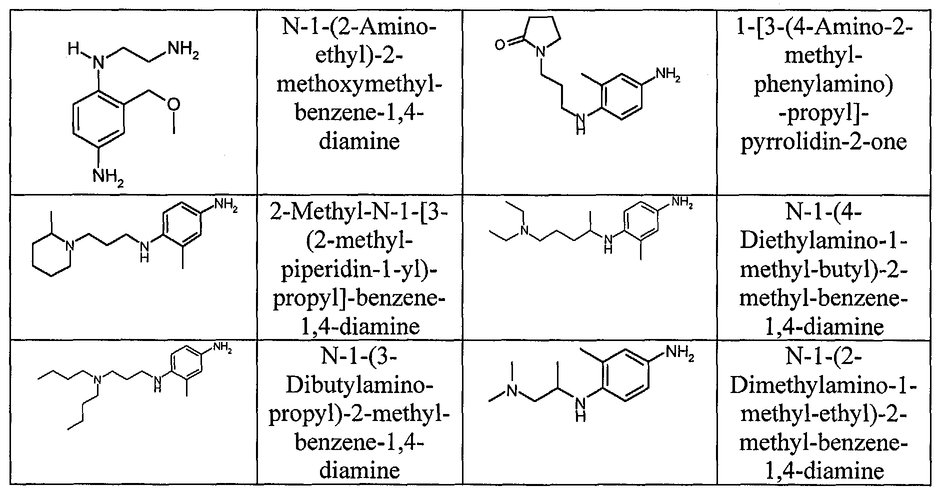

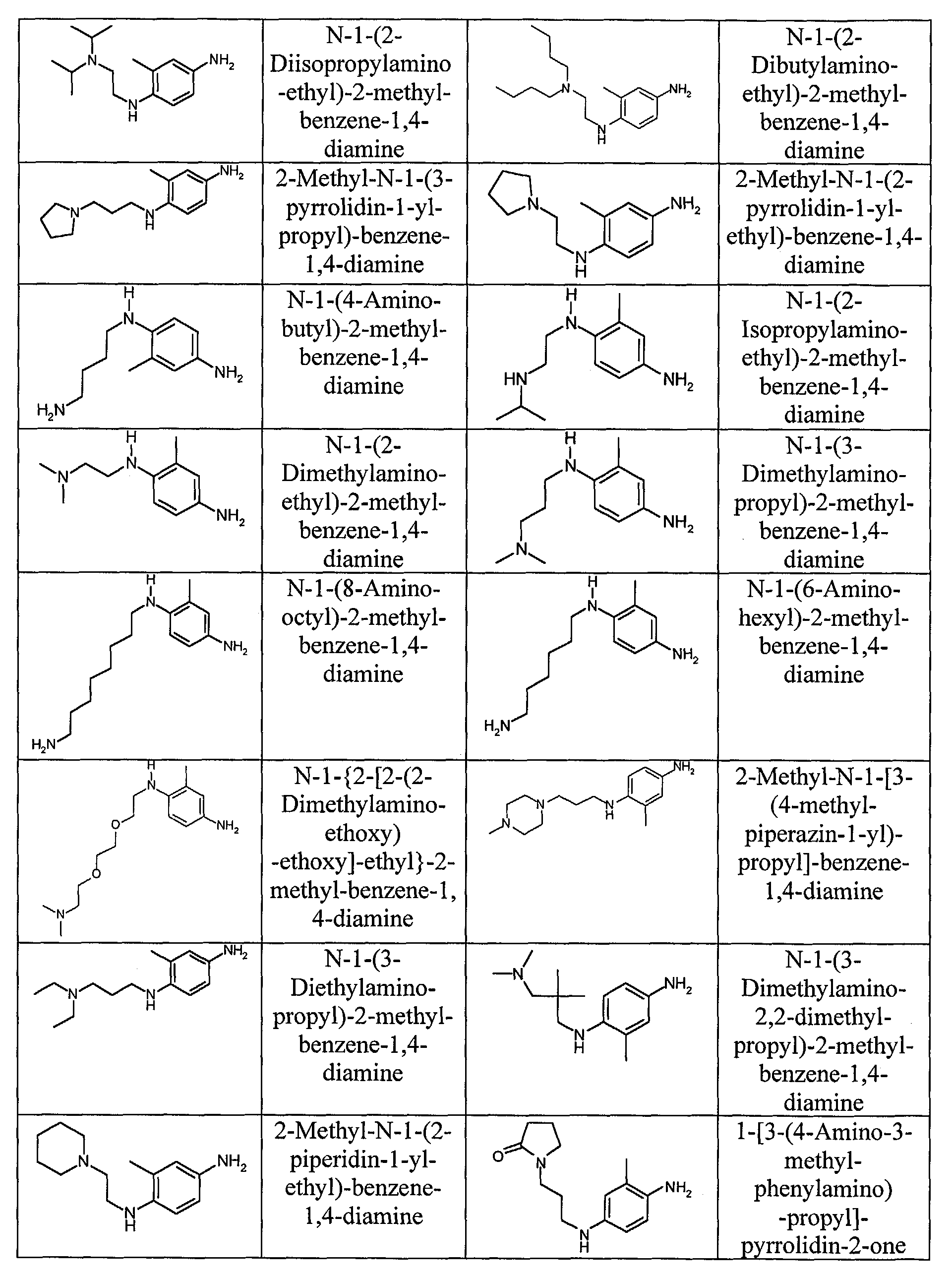

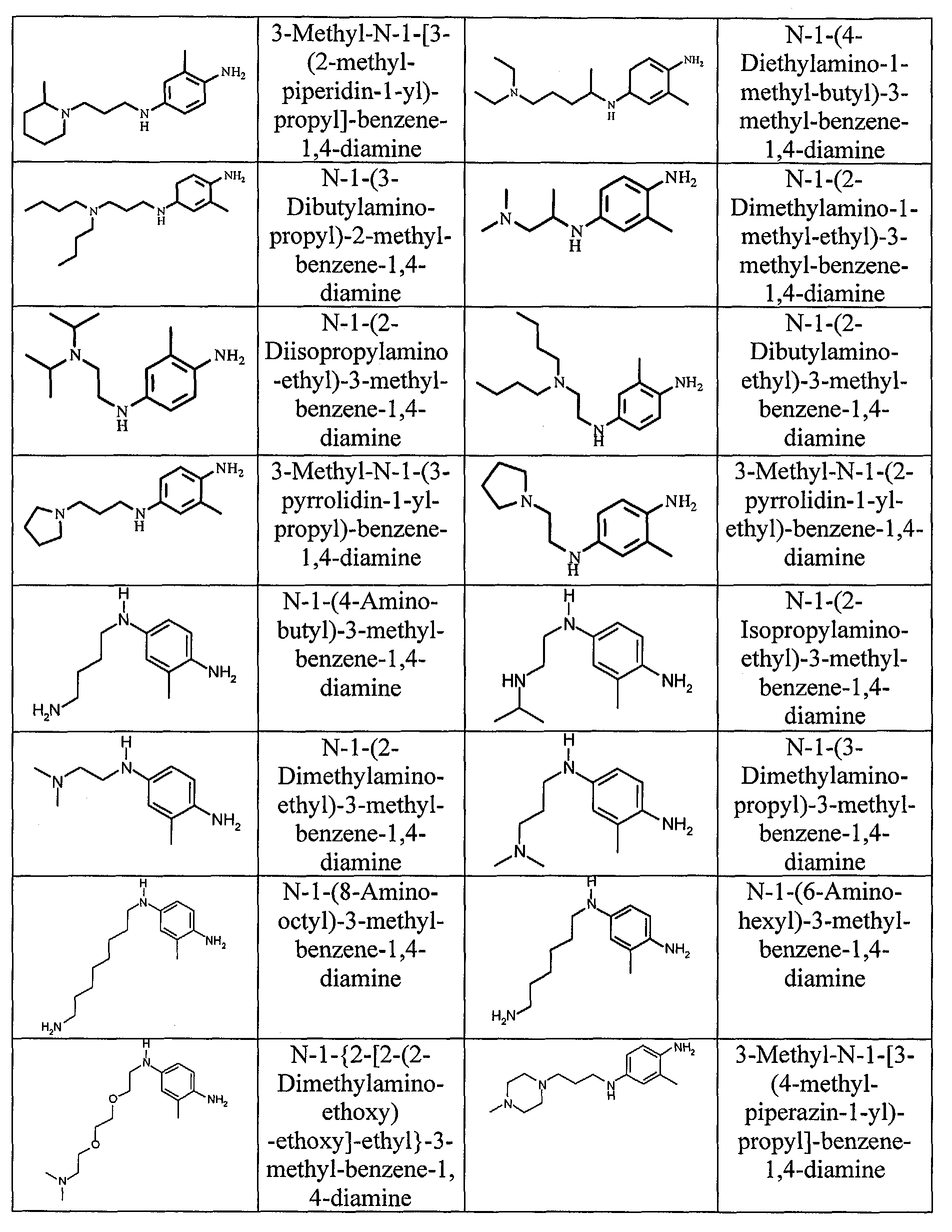

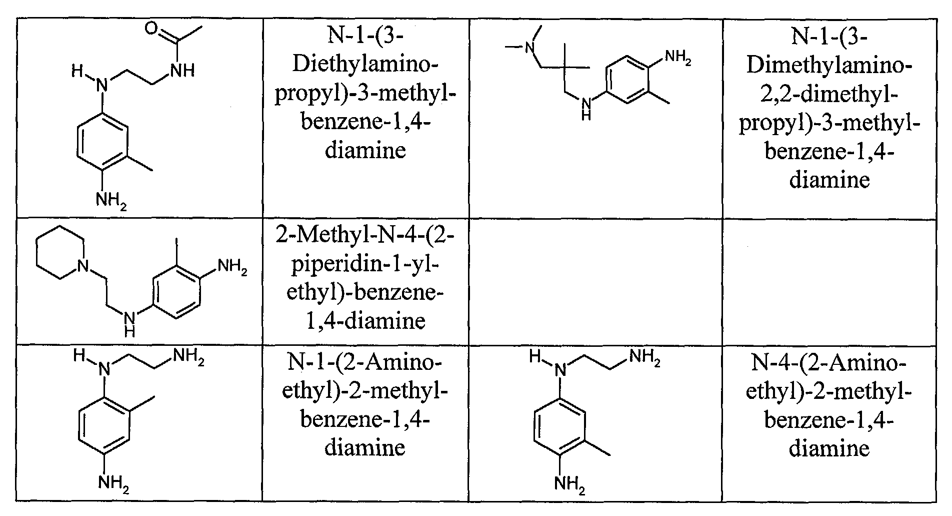

Les composés (I) selon la présente demande peuvent être préparés de façon générale suivant une méthode qui comprend les étapes suivantes :
- synthèse d'un composé 4(N-alkylaminé)nitrobenzène par substitution nucléophile de l'halogène d'un para-halogéno nitrobenzène par une diamine de formule R₁R₂NRNH₂ (R₁, R₂ et R étant tels que définis ci-dessus) en présence d'une base,
- réduction du groupement nitro du composé 4(N-alkylaminé)nitrobenzène obtenu pour obtenir le composé de formule (I) :

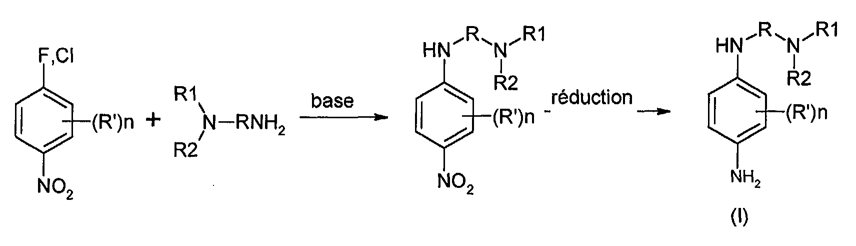

La première étape de synthèse est décrite dans les documents Synthesis 1990 (12), 1147-1148 et Synth Commun 1990, 20(22), 3537-3543.

La deuxième étape est une étape de réduction classique par exemple en effectuant une réaction d'hydrogénation par catalyse hétérogène en présence de Pd/C, Pd(II)/C, Ni Raney... ou encore en effectuant une réaction de réduction par un métal, par exemple par du zinc, fer, étain.. (Advanced Organic Chemistry, 4th edition, 1992, J.MARCH, WILEY Interscience ; Reduction in Organic Chemistry, M.Hudlicky, 1983, Ellis Honwood series Chemical Science).

### Une deuxième voie de synthèse peut être schématisée comme suit :

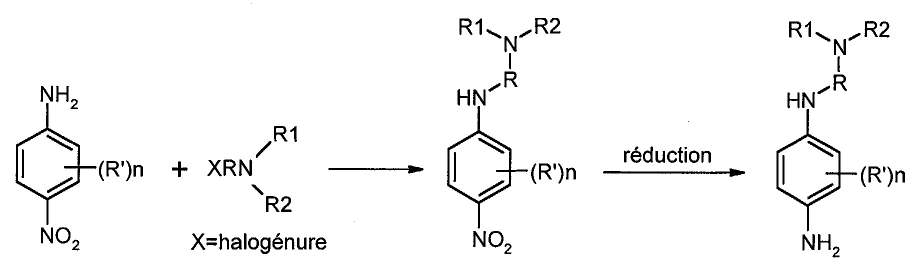

1^{ére} étape : étape de condensation s'inspirant du J.Indian. Chem. Soc. 1990, 67, 602-603 ou du Synth. Commun.1999, 29, 1819-1933
2^{ème} étape : étape de réduction classique qui est effectuée comme exposé dans la méthode générale ci-dessus.

La présente demande concerne aussi les composés nitrés de formule (II) : 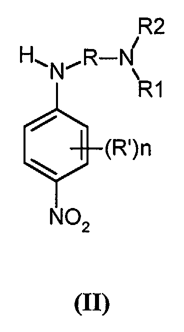 dans laquelle :
les radicaux R₁ et R₂ représentent, indépendamment l'un de l'autre, un atome d'hydrogène, un groupement alkyle, ou les radicaux R₁ et R₂ forment, ensemble et avec l'azote qu'ils substituent, un cycle saturé à 5 ou 6 chaînons éventuellement interrompu par un ou plusieurs atomes d'azote et/ou par un ou plusieurs groupements carbonyle, et éventuellement substitué par un ou plusieurs groupements alkyles,
le radical R représente un radical alkylène en C₂-C₁₀, linéaire ou ramifié, éventuellement interrompu par un ou plusieurs atomes choisis parmi l'azote ou l'oxygène et éventuellement substitué par un ou plusieurs groupements choisis parmi les groupements amino, monoalkylamino, dialkylamino, alkylcarbonyle, amido, alkoxycarbonyle, monoalkylaminocarbonyle, dialkylaminocarbonyle,
le radical R' représente un radical choisi parmi les radicaux alkyle, alcoxy, alcoxyalkyle, monohydroxyalkyle , polyhydroxyalkyle et l'atome de chlore,
n est un entier égal à 1 ; à l'exclusion des N-1(3-diethylaminopropyl)-3-methyl-4-nitro-1-aminobenzene et N-1-(3-pyrrolidino-propyl)3-methyl-4-nitro-1-aminobenzene.
   et les procédés de préparation des composés para-phénylènediamines secondaires N-alkylaminées ortho substituées et/ou méta substituées de formule (I) dans lequel on effectue une étape de réduction du composé nitré correspondant ; par « composé nitré correspondant » on entend le composé de formule (I) dans lequel le groupe amino en para du groupe NHRNR₁R₂ est remplacé par un groupe nitro.

La présente demande concerne également l'utilisation, pour la teinture des fibres kératiniques, de préférence les fibres kératiniques humaines telles que les cheveux, d'un composé de formule (I), c'est-à-dire un composé de formule (I) 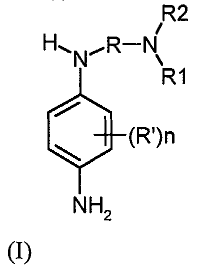 dans laquelle :
les radicaux R₁ et R₂ représentent, indépendamment l'un de l'autre, un atome d'hydrogène, un groupement alkyle, ou les radicaux R₁ et R₂ forment, ensemble et avec l'azote qu'ils substituent, un cycle saturé à 5 ou 6 chaînons éventuellement interrompu par un ou plusieurs atomes d'azote et/ou par un ou plusieurs groupements carbonyle, et éventuellement substitué par un ou plusieurs groupements alkyles,
le radical R représente un radical alkylène en C₂-C₁₀, linéaire ou ramifié, éventuellement interrompu par un ou plusieurs atomes choisis parmi l'azote ou l'oxygène, et éventuellement substitué par un ou plusieurs groupements choisis parmi les groupements amino,
monoalkylamino, dialkylamino, alkylcarbonyle, amido, alkoxycarbonyle, monoalkylaminocarbonyle, dialkylaminocarbonyle,
le radical R' représente un radical choisi parmi les radicaux alkyle, alcoxy, alcoxyalkyle, monohydroxyalkyle , polyhydroxyalkyle et l'atome de chlore,
n est un entier égal à 1.

La présente demande concerne aussi une composition cosmétique pour la teinture des fibres kératiniques, de préférence les fibres kératiniques humaines telles que les cheveux, comprenant, dans un milieu approprié pour la teinture, au moins un composé de formule générale (I).

La présente demande concerne aussi une composition cosmétique pour la teinture des fibres kératiniques, de préférence les fibres kératiniques humaines telles que les cheveux, comprenant, dans un milieu approprié pour la teinture, au moins un composé de formule générale (I) et au moins un adjuvant cosmétique choisi dans le groupe formé par les agents anti-oxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

La présente demande concerne également l'utilisation d'une composition cosmétique comprenant, dans un milieu approprié pour la teinture, au moins un composé de formule générale (I), pour la teinture des fibres, de préférence les fibres kératiniques telles que les cheveux.

De préférence, la concentration du composé de formule générale (I) est comprise entre 0,0001 et 20%, de préférence entre 0,005 à 6% en poids par rapport au poids total de la composition.

Le milieu approprié pour la teinture est avantageusement constitué par de l'eau ou par un mélange d'eau et d'au moins un solvant organique tel que, par exemple, les alcools inférieurs en C₁-C₄, ramifiés ou non, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, le glycérol ainsi que les alcools aromatiques comme l'alcool benzylique ou le phénoxyéthanol, et leurs mélanges.

Avantageusement, la composition cosmétique comprend au moins un adjuvant cosmétique choisi dans le groupe formé par les agents anti-oxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

Les adjuvants ci-dessus sont en général présents en quantité comprise pour chacun d'eux entre 0,01 et 20 % en poids par rapport au poids de la composition.

La composition selon l'invention contient de préférence au moins un précurseur de colorant d'oxydation additionnel différent des composés de formule (I), de manière encore plus préférée la composition selon l'invention contient au moins un coupleur.

Parmi les coupleurs d'oxydation, on peut notamment citer les méta-phénylènediamines, les méta-aminophénols, les méta-diphénols, les coupleurs naphtaléniques et les coupleurs hétérocycliques ainsi que leurs sels d'addition.

A titre d'exemple, on peut citer le 2-méthyl 5-aminophénol, le 5-N-(ß-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine, la 3,5-diamino-2,6-diméthoxypyridine, le 1-N-(ß-hydroxyéthyl)amino-3,4-méthylène dioxybenzène, le 2,6-bis-(ß-hydroxyéthylamino)toluène et leurs sels d'addition.

Généralement la concentration du ou des coupleurs d'oxydation est comprise entre 0,0001 et 20%, de préférence entre 0,005 à 6% en poids par rapport au poids total de la composition.

Les bases d'oxydation additionnelles différentes des composés de formule (I) peuvent notamment être choisies parmi les para-phénylènediamines, les bis-phénylalkylènediamines, les para--aminophénols, les ortho-aminophénols et les bases hétérocycliques et leurs sels d'addition.

Parmi les para-phénylènediamines, on peut plus particulièrement citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4 aminophenyl pyrrolidine, le 2 thiényl para-phénylène diamine, le 2-β hydroxyéthylamino 5-amino toluène, la 3-hydroxy 1-(4' aminophenyl)pyrrolidine, la 6-(4-Amino-phenylamino)-hexan-1-ol et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para--aminophénol, le 4-amino 3-méthyl phénol, le 4-amino 3-fluoro phénol, le 4-amino 2-chloro phénol, le 4-amino 3-chloro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino-2,6 dichlorophénol, le 4-amino 6[((5'amino-2'hydroxy-3'-méthyl)phényl)méthyl]-2-méthylphénol, le bis[(5'amino-2'hydroxy)phénylméthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ;
ainsi que leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Généralement la concentration de la ou les bases d'oxydation additionnelle(s) est comprise entre 0,0001 et 20%, de préférence entre 0,005 à 6% en poids par rapport au poids total de la composition.

D'une manière générale, les sels d'addition avec un acide utilisables pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

La composition tinctoriale conforme à l'invention peut en outre contenir un ou plusieurs colorants directs pouvant notamment être choisis parmi les colorants nitrés de la série benzénique, neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs méthiniques, azométhiniques, triarylméthaniques, indoaminiques et les colorants directs naturels. De préférence, la composition selon l'invention comprend au moins un colorant choisi parmi les colorants directs cationiques et les colorants directs naturels.

Parmi les colorants directs cationiques utilisables selon l'invention on peut citer les colorants directs azoïques cationiques décrits dans les demandes de brevets WO 95/15144, WO-95/01772 et EP-714954.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono) méthyl]-pyridinium.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

Bien entendu, l'homme de l'art veillera à choisir le ou les adjuvants, précurseurs de colorants d'oxydations additionnels, colorants directs de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques autres que les diacides carboxyliques comme l'acide chlorhydrique, l'acide ortho-phosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule : 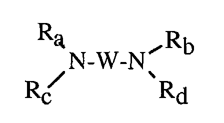 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition cosmétique selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La présente demande concerne un procédé dans lequel on applique sur les fibres kératiniques la composition selon la présente invention telle que définie précédemment pendant une durée suffisante pour développer la coloration désirée en présence d'un agent oxydant, l'agent oxydant étant appliqué avant, simultanément ou après la composition. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration : selon ce mode de réalisation particulier, on dispose d'une composition prête à l'emploi qui est un mélange d'une composition selon l'invention avec au moins un agent oxydant de préférence choisi parmi le peroxyde d'hydrogène, le peroxyde d'urée, les bromates de métaux alcalins, les persels, les peracides et les enzymes oxydases. Le mélange obtenu, sous la forme d'une composition prête à l'emploi, est ensuite appliqué sur les fibres kératiniques pendant une durée suffisante pour développer la coloration désirée. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour développer la coloration désirée.

Les exemples qui suivent illustrent l'invention sans en limiter la portée.

### EXEMPLES

### Exemple 1 : Synthèse du N-1-(2-amino-éthyl)-2-méthoxyméthyl-benzène-1,4-diamine, trichlorhydrate (25)

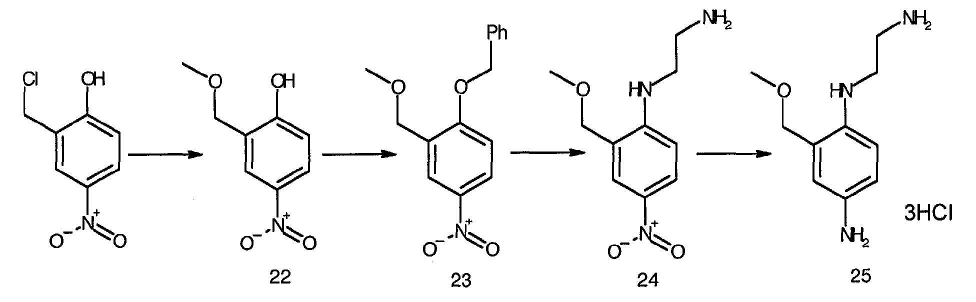

### Etape 1 : Préparation du 2-Méthoxyméthyl-4-nitro-phénol (22)

18,7g (0,1 mole) de chlorométhyl-2-nitro-4-phénol et 50ml de méthanol sont portés au reflux 2h30. A cette solution on additionne goutte à goutte 37ml (0,2 mole) de méthylate de sodium 5,4N. Après refroidissement le milieu réactionnel est versé dans 350ml de glace-eau. On filtre un léger insoluble puis acidifie le filtrat avec 10 ml d'acide acétique ; l'huile cristallise. On obtient 11,4g de 2-Méthoxyméthyl-4-nitro-phénol.

### Etape 2 : Préparation du 1-Benzyloxy-2-méthoxyméthyl-4-nitro-benzène (23)

A un mélange de 18,3g (0,1mole) de 2-Méthoxyméthyl-4-nitro-phénol (22) obtenu précédemment, 8,3g (0,06mole) de carbonate de potassium dans 40ml de diméthylformamide chauffés au bain-marie bouillant, on coule en 10 minutes, 12,1ml (0,105mole) de chlorure de benzyle. Le mélange réactionnel est versé sur 150g de glace. Les cristaux jaunes obtenus sont filtrés puis lavés à l'eau et recristallisés dans l'isopropanol. On obtient 21,1g (Fusion = 108°C).

| | **THEORIE** | **TROUVE** |
|---|---|---|
| C | 65.93 | 65.86 |
| H | 5.53 | 5.56 |
| O | 23.42 | 23.62 |
| N | 5.13 | 5.19 |

### Etape 3 : Préparation du N-1-(2-Méthoxyméthyl-4-nitro-phényl)-éthane-1,2-diamine (24)

A 150ml d'éthylène diamine, chauffés au bain-marie bouillant, on ajoute par portions en 10 minutes 54,6g (0,2mole) de 1-Benzyloxy-2-méthoxyméthyl-4-nitrobenzène (23). Après 4h30 de chauffage, on verse le milieu réactionnel dans 800g de glace-eau. Le précipité obtenu est essoré et séché. On obtient alors 31,2g de produit.

| | **THEORIE** | **TROUVE** |
|---|---|---|
| C | 53.32 | 53.33 |
| H | 6.71 | 6.72 |
| O | 21.31 | 21.57 |
| N | 18.65 | 18.54 |

### Etape 4 : Préparation du N-1-(2-amino-éthyl)-2-méthoxyméthyl-benzène-1,4-diamine, trichlorhydrate (25)

27,15g (0,12mole) de N-1-(2-Méthoxyméthyl-4-nitro-phényl)-éthane-1,2-diamine sont réduits dans 60g de zinc en poudre, 12ml d'eau, 120ml d'éthanol et 2,4g de chlorure d'ammonium portés au reflux. Après filtration, on piège la para-phénylènediamine sous forme de chlorhydrate par addition de 70ml d'éthanol chlorhydrique 7N. Par évaporation à sec, on obtient 31g de N-1-(2-amino-éthyl)-2-méthoxyméthyl-benzène-1,4-diamine.

| | **THEORIE** | **TROUVE** |
|---|---|---|
| C | 39.43 | 53.33 |
| H | 6.62 | 6.72 |
| O | 34.91 | 21.57 |
| N | 13.79 | 18.54 |
| Cl | 34.91 | 35.35 |

Les spectres de RMN du proton et de masse sont conformes à la structure attendue du produit.

### Exemple 2 : Synthèse du 1-[3-(4-Amino-3-methyl-phenylamino)-propyl]-pyrrolidin-2-one, dichlorhydrate (27)

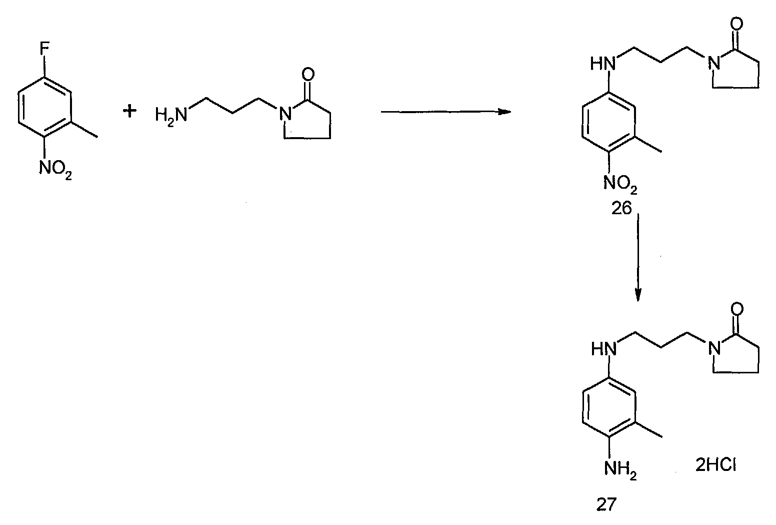

### Etape 1 : synthèse du 1-[3-(4-nitro-3-methyl-phenylamino)-propyl]-pyrrolidin-2-one (26)

A une solution de 20 ml de N-méthyl-pyrrolidinone, 2,2 g de N-(3'aminopropyl) -2-pyrrolidinone et 2,14 g de K₂CO₃, on ajoute 2 g de 5-fluoro-2-nitrotoluène. Le milieu réactionnel est chauffé à 60°C pendant 15 heures, puis, après refroidissement à la température ambiante, est versé dans un mélange eau + glace. Le précipité jaune formé est filtré, ré-empaté dans l'eau puis séché sur P₂O₅. On obtient 2,8 g de 1-[3-(4-nitro-3-methyl-phenylamino)-propyl]-pyrrolidin-2-one (26).

### Etape 2 : synthèse du 1-[3-(4-Amino-3-methyl-phenylamino)-propyl]-pyrrolidin-2-one dichlorhydrate (27)

Le 1-[3-(4-nitro-3-methyl-phenylamino)-propyl]-pyrrolidin-2-one (26) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 3: Synthèse du N-1-(2-Dimethylamino-ethyl)-2-methyl-benzene-1,4-diamine, dichlorhydrate (29)

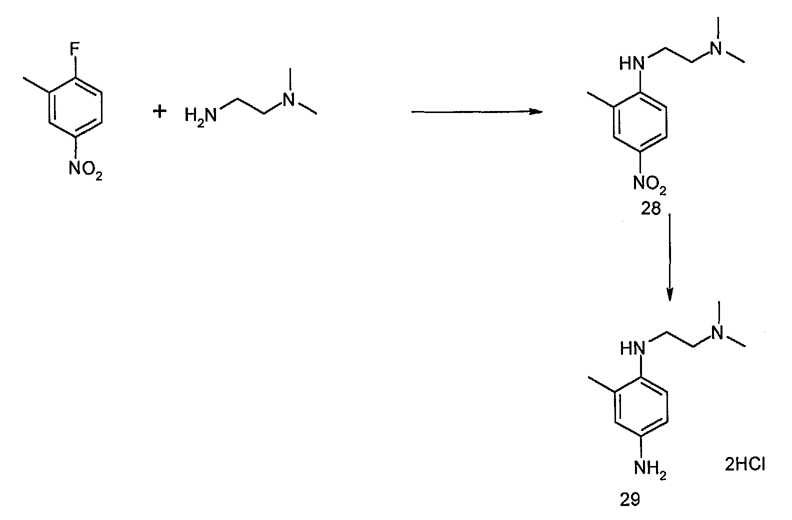

### Etape 1 : synthèse du N-1-(2-Dimethylamino-ethyl)-2-methyl-4-nitro-1-aminobenzene (28)

A une solution de 20 ml de N-méthyl-pyrrolidinone, 1,36 g de 2-diméthylaminoéthylamine et 2,14 g de K₂CO₃ , on ajoute 2 g de 2-fluoro-5-nitrotoluène. Le milieu réactionnel est chauffé à 60°C pendant 12 heures, puis, après refroidissement à la température ambiante, est versé dans un mélange eau + glace. Le précipité jaune formé est filtré, ré-empaté dans l'eau puis séché sur P₂O₅. On obtient 2,3 g de N-1-(2-Dimethylamino-ethyl)-2-methyl-4-nitro-1-aminobenzene (28).

### Etape 2 : synthèse du N-1-(2-Dimethylamino-ethyl)-2-methyl-benzene-1,4-diamine, dichlorhydrate (29)

Le N-1-(2-Dimethylamino-ethyl)-2-methyl-4-nitro-1-aminobenzene (28) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 4: Synthèse du 2-Methyl-N-4-(2-pyrrolidin-1-yl-ethyl)-benzene-1,4-diamine, dichlorhydrate (31)

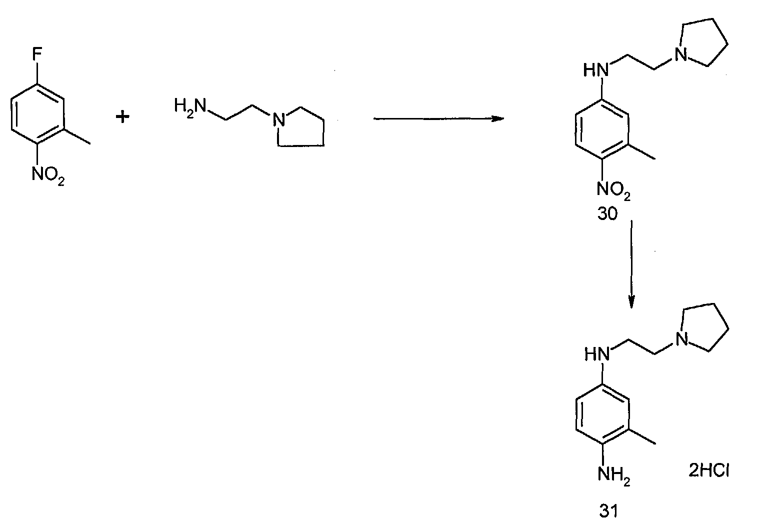

### Etape 1 : synthèse du 2-Methyl-N-4-(2-pyrrolidin-1-yl-ethyl)-4-nitro-1-aminobenzene (30)

A une solution de 20 ml de N-méthyl-pyrrolidinone, 1,76 g de 2-(1-pyrrolidino)éthylamine et 1,57 g de triéthylamine , on ajoute 2 g de 5-fluoro-2-nitrotoluène. Le milieu réactionnel est chauffé à 60°C pendant 12 heures, puis, après refroidissement à la température ambiante, est versé dans un mélange eau + glace. Le milieu résultant est extrait à l'acétate d'éthyle puis la phase organique est concentrée sous pression réduite. On obtient 3,2 g de 2-Methyl-N-4-(2-pyrrolidin-1-yl-ethyl)-4-nitro-1-aminobenzene (30).

### Etape 2 : synthèse du 2-Methyl-N-4-(2-pyrrolidin-1-yl-ethyl)-benzene-1,4-diamine, dichlorhydrate (31)

Le 2-Methyl-N-4-(2-pyrrolidin-1-yl-ethyl)-4-nitro-1-aminobenzene (30) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 5: Synthèse du N-1-(3-Diethylamino-propyl)-2-methyl-benzene-1,4-diamine, dichlorhydrate (33)

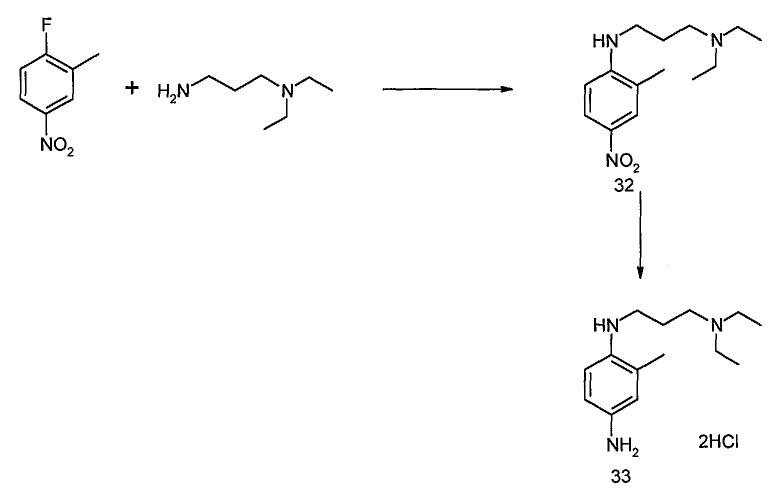

### Etape 1 : synthèse du N-1-(3-Diethylamino-propyl)-2-methyl-4-nitro-1-aminobenzene (32)

A une solution de 20 ml de N-méthyl-pyrrolidinone, 2,01 g de 3-diethylamino-propylamine et 2,14 g de K₂CO₃ , on ajoute 2 g de 2-fluoro-5-nitrotoluène. Le milieu réactionnel est chauffé à 60°C pendant 12 heures, puis, après refroidissement à la température ambiante, est versé dans un mélange eau + glace. Le précipité jaune formé est filtré, ré-empaté dans l'eau puis séché sur P₂O₅. On obtient 3,35 g de N-1-(3-Diethylamino-propyl)-2-methyl-4-nitro-1-aminobenzene (32).

### Etape 2 : synthèse du N-1-(3-Diethylamino-propyl)-2-methyl-benzene-1,4-diamine dichlorhydrate (33)

Le N-1-(3-Diethylamino-propyl)-2-methyl-4-nitro-1-aminobenzene (32) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 6 : Synthèse du N-1-(3-dibutylamino-propyl)-2-methyl-benzene-1,4-diamine, dichlorhydrate (35)

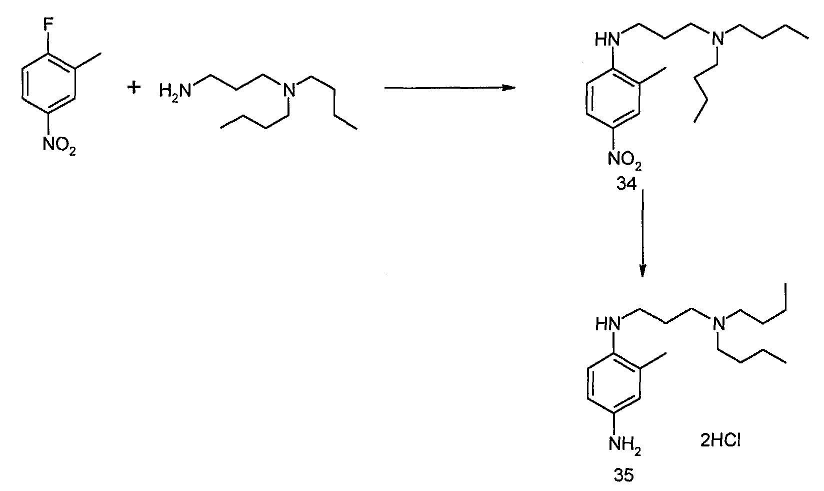

### Etape 1 : synthèse du N-1-(3-dibutylamino-propyl)-2-methyl-4-nitro-1-aminobenzene (34)

A une solution de 20 ml de N-méthyl-pyrrolidinone, 2,88 g de 3-dibutylamino-propylamine et 1,57 g g de triéthylamine , on ajoute 2 g de 2-fluoro-5-nitrotoluène. Le milieu réactionnel est chauffé à 60°C pendant 15 heures, puis, après refroidissement à la température ambiante, est versé dans un mélange eau + glace. Le milieu réactionnel est extrait à l'acétate d'éthyle puis la phase organique est concentrée sous pression réduite. On obtient 0,74 g de N-1-(3-dibutylamino-propyl)-2-methyl-4-nitro-1-aminobenzene (34).

### Etape 2 : synthèse du N-1-(3-dibutylamino-propyl)-2-methyl-benzene-1,4-diamine dichlorhydrate (35)

L e N-1-(3-dibutylamino-propyl)-2-methyl-4-nitro-1-aminobenzene (34) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 7 : Synthèse du N-1-(3-dimethylamino-propyl)-2-methyl-benzene-1,4-diamine, dichlorhydrate (37)

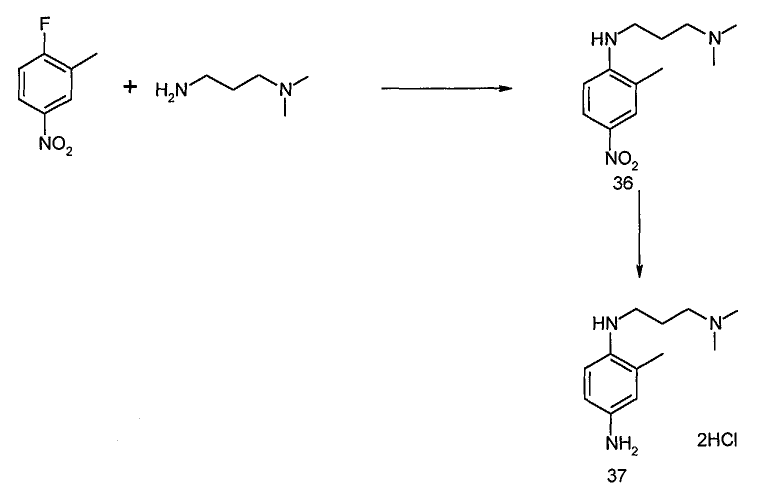

### Etape 1 : synthèse du N-1-(3-dimethylamino-propyl)-2-methyl-4-nitro-1-aminobenzene (36)

A une solution de 20 ml de N-méthyl-pyrrolidinone, 1,58 g de N,N-diméthyl-1,3-diaminopropane et 2,14 g de K₂CO₃ , on ajoute 2 g de 2-fluoro-5-nitrotoluène. Le milieu réactionnel est chauffé à 60°C pendant 12 heures, puis, après refroidissement à la température ambiante, est versé dans un mélange eau + glace. Le précipité jaune formé est filtré, ré-empaté dans l'eau puis séché sur P₂O₅. On obtient 3 g de N-1-(3-dimethylamino-propyl)-2-methyl-4-nitro-1-aminobenzene (36).

### Etape 2: synthèse du N-1-(3-dimethylamino-propyl)-2-methyl-benzene-1,4-diamine dichlorhydrate (37).

Le N-1-(3-dimethylamino-propyl)-2-methyl-4-nitro-1-aminobenzene (36) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 8 : Synthèse du N-1-(2-Diisopropylamino-ethyl)-2-methyl-benzene-1,4-diamine, dichlorhydrate (39)

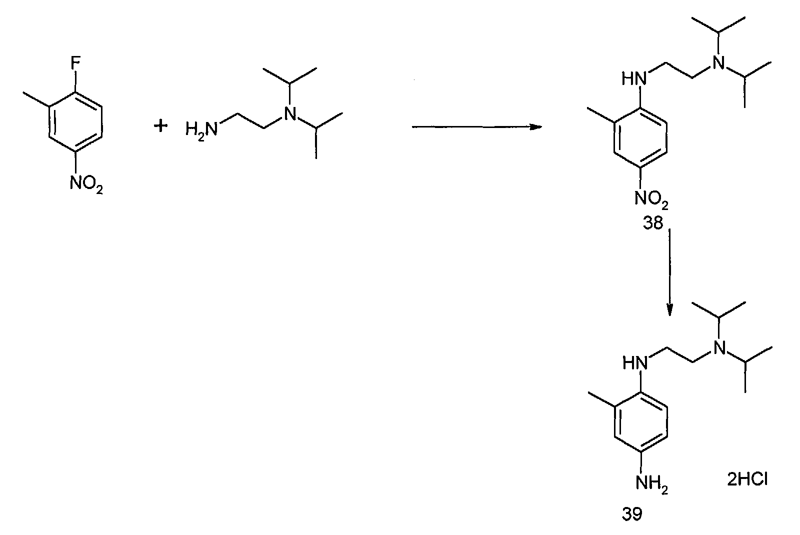

### Etape 1 : synthèse du N-1-(2-Diisopropylamino-ethyl)-2-methyl-4-nitro-1-aminobenzene (38).

A une solution de 20 ml de N-méthyl-pyrrolidinone, 2,23 g de N,N-diisopropyléthylènediamine et 1,57 g de triéthylamine, on ajoute 2 g de 2-fluoro-5-nitrotoluène. Le milieu réactionnel est chauffé à 60°C pendant 12 heures, puis, après refroidissement à la température ambiante, est versé dans un mélange eau + glace. Le précipité jaune formé est filtré, ré-empaté dans l'eau puis séché sur P₂O₅. On obtient 3,3 g de N-1-(2-Diisopropylamino-ethyl)-2-methyl-4-nitro-1-aminobenzene (38).

### Etape 2 : synthèse du N-1-(2-Diisopropylamino-ethyl)-2-methyl-benzene-1,4-diamine dichlorhydrate (39)

Le N-1-(2-Diisopropylamino-ethyl)-2-methyl-4-nitro-1-aminobenzene (38) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 9 : Synthèse du 2-Methyl-N-1-(3-pyrrolidin-1-yl-propyl)-benzene-1,4-diamine, dichlorhydrate (41)

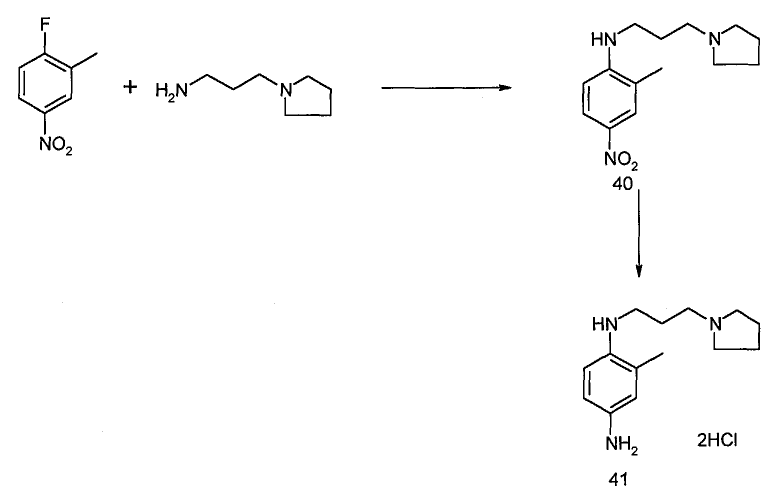

### Etape 1 : synthèse du 2-Methyl-N-1-(3-pyrrolidin-1-yl-propyl)-4-nitro-1-aminobenzene (40)

A une solution de 20 ml de N-méthyl-pyrrolidinone, 1,98 g de N-(3-aminopropyl)pyrrolidine et 2,14 g de K₂CO₃ , on ajoute 2 g de 2-fluoro-5-nitrotoluène. Le milieu réactionnel est chauffé à 60°C pendant 15 heures, puis, après refroidissement à la température ambiante, est versé dans un mélange eau + glace. Le précipité jaune formé est filtré, ré-empaté dans l'eau puis séché sur P₂O₅. On obtient 3,2 g de 2-Methyl-N-1-(3-pyrrolidin-1-yl-propyl)-4-nitro-1-aminobenzene (40).

### Etape 2 : synthèse du 2-Methyl-N-1-(3-pyrrolidin-1-yl-propyl)-benzene-1,4-diamine, dichlorhydrate (41)

Le 2-Methyl-N-1-(3-pyrrolidin-1-yl-propyl)-4-nitro-1-aminobenzene (40) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 10 : Synthèse du 3-Methyl-N-1-(3-pyrrolidin-1-yl-propyl)-benzene-1,4-diamine, dichlorhydrate (43)

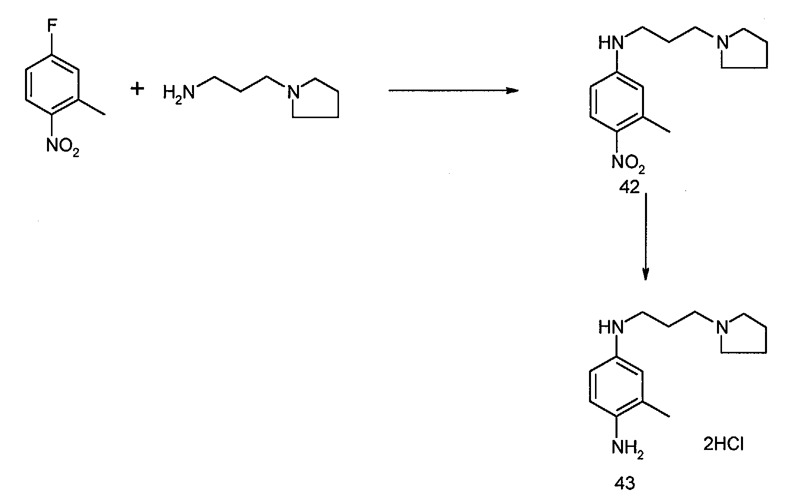

### Etape 1 : synthèse du 3-Methyl-N-1-(3-pyrrolidin-1-yl-propyl)-4-nitro-1-aminobenzene (42).

A une solution de 20 ml de N-méthyl-pyrrolidinone, 1,98 g de (N-3-aminopropyl)pyrrolidine et 1,57 g de triéthylamine , on ajoute 2 g de 5-fluoro-2-nitrotoluène. Le milieu réactionnel est chauffé à 60°C pendant 12 heures, puis, après refroidissement à la température ambiante, est versé dans un mélange eau + glace. Le milieu résultant est extrait au dichlorométhane puis la phase organique est concentrée sous pression réduite. On obtient 3,39 g de 3-Methyl-N-1-(3-pyrrolidin-1-yl-propyl)-4-nitro-1-aminobenzene (42).

### Etape 2 : synthèse du 3-Methyl-N-1-(3-pyrrolidin-1-yl-propyl)-benzene-1,4-diamine, dichlorhydrate

Le 3-Methyl-N-1-(3-pyrrolidin-1-yl-propyl)-4-nitro-1-aminobenzene précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 11 : Synthèse du 1-[3-(4-Amino-2-methyl-phenylamino)-propyl]-pyrrolidin-2-one, dichlorhydrate (45)

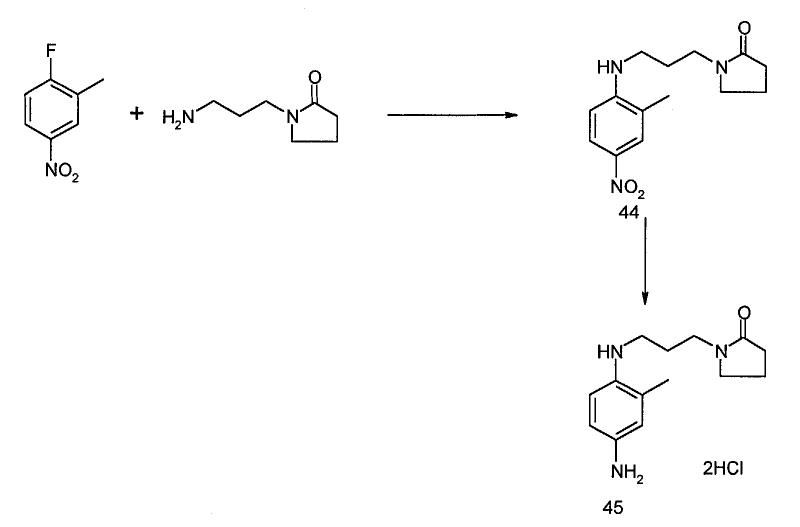

### Etape 1 : synthèse du 1-[3-(4-nitro-3-methyl-phenylamino)-propyl]-pyrrolidin-2-one (44)

A une solution de 20 ml de N-méthyl-pyrrolidinone, 0,41 g de N-(3'aminopropyl) -2-pyrrolidinone et 0,29 g de triethylamine , on ajoute 2 g de 2-fluoro-5-nitrotoluène. Le milieu réactionnel est chauffé à 60°C pendant 15 heures, puis, après refroidissement à la température ambiante, est versé dans un mélange eau + glace. Le précipité jaune formé est filtré, ré-empaté dans l'eau puis séché sur P₂O₅. On obtient 0,38 g de 1-[3-(4-nitro-3-methyl-phenylamino)-propyl]-pyrrolidin-2-one (44).

### Etape 2 : synthèse du 1-[3-(4-Amino-3-methyl-phenylamino)-propyl]-pyrrolidin-2-one dichlorhydrate (45)

Le 1-[3-(4-nitro-3-methyl-phenylamino)-propyl]-pyrrolidin-2-one (44) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 12: Synthèse du 3-Methyl-N-4-(2-pyrrolidin-1-yl-ethyl)-benzene-1,4-diamine, dichlorhydrate (47)

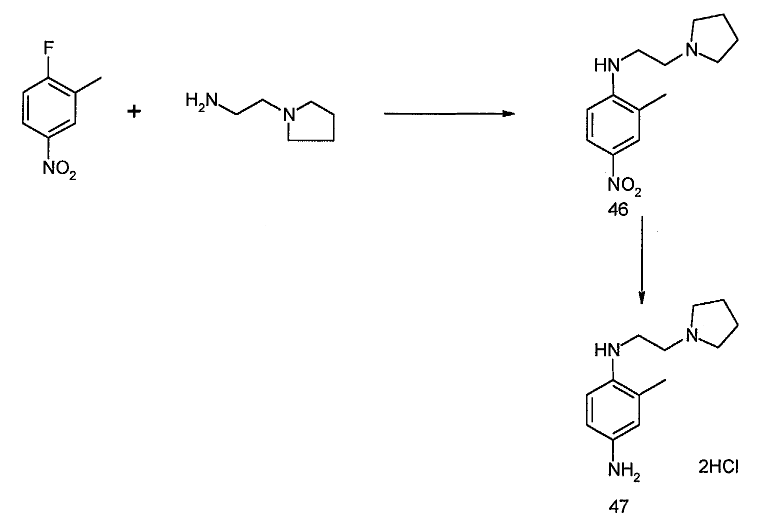

### Etape 1 : synthèse du 3-Methyl-N-4-(2-pyrrolidin-l-yl-ethyl)-4-nitro-1-aminobenzene (46)

A une solution de 20 ml de N-méthyl-pyrrolidinone, 1,76 g de N-(3-aminoethyl)pyrrolidine et 1,56 g de triethylamine , on ajoute 2 g de 2-fluoro-5-nitrotoluène. Le milieu réactionnel est chauffé à 60°C pendant 15 heures, puis, après refroidissement à la température ambiante, est versé dans un mélange eau + glace. Le précipité jaune formé est filtré, ré-empaté dans l'eau puis séché sur P₂O₅. On obtient 2 ,8g de 3-Methyl-N-4-(2-pyrrolidin-1-yl-ethyl)-4-nitro-1-aminobenzene (46).

### Etape 2 : synthèse du 3-Methyl-N-4-(2-pyrrolidin-1-yl-ethyl)-benzene-1,4-diamine, dichlorhydrate (47)

Le 3-Methyl-N-4-(2-pyrrolidin-1-yl-ethyl)-4-nitro-1-aminobenzene (46) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 13 : Synthèse du N-(2-aminoethyl)-N-(4-amino-3-methoxyphenyl)amine, dichlorhydrate (49)

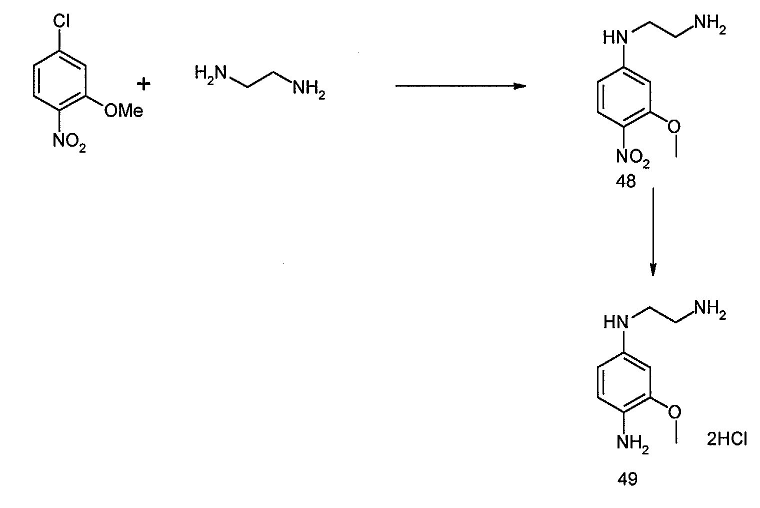

Le 5-chloro-2-nitroanisole (0.2 mmol dans 500µL de NMP-EtOH), l'éthylènediamine (0.24 mmol dans 500µL de NMP) et la diisopropylethylamine (0.48mmol) sont chauffés à 110°C pendant 24 heures. Le milieu réactionnel est ensuite refroidi et concentré sous vide. Le résidu est repris dans 2 mL d'éthanol et le cyclohéxène (1 mL) est ajouté ainsi qu'une suspension de 50 mg de charbon palladié à 5% dans 500µL d'éthanol. Ce mélange est chauffé à 80°C pendant 3 heures. Le milieu réactionnel est alors filtré et traité par une solution d'éthanol chlorhydrique. La solution est ensuite concentré pour fournir le composé désiré sous la forme dichlorhydrate.

Les spectres RMN du proton et de masse sont conformes à la structure attendue du produit.

### Exemples 45 à 55 : Composition tinctoriale à partir du N-1-(2-amino-éthyl)-2-méthoxyméthyl-benzène-1,4-diamine, trichlorhydrate (25)

### -Exemples 45 à 51 : Teinture en milieu acide

Les compositions tinctoriales suivantes sont préparées :

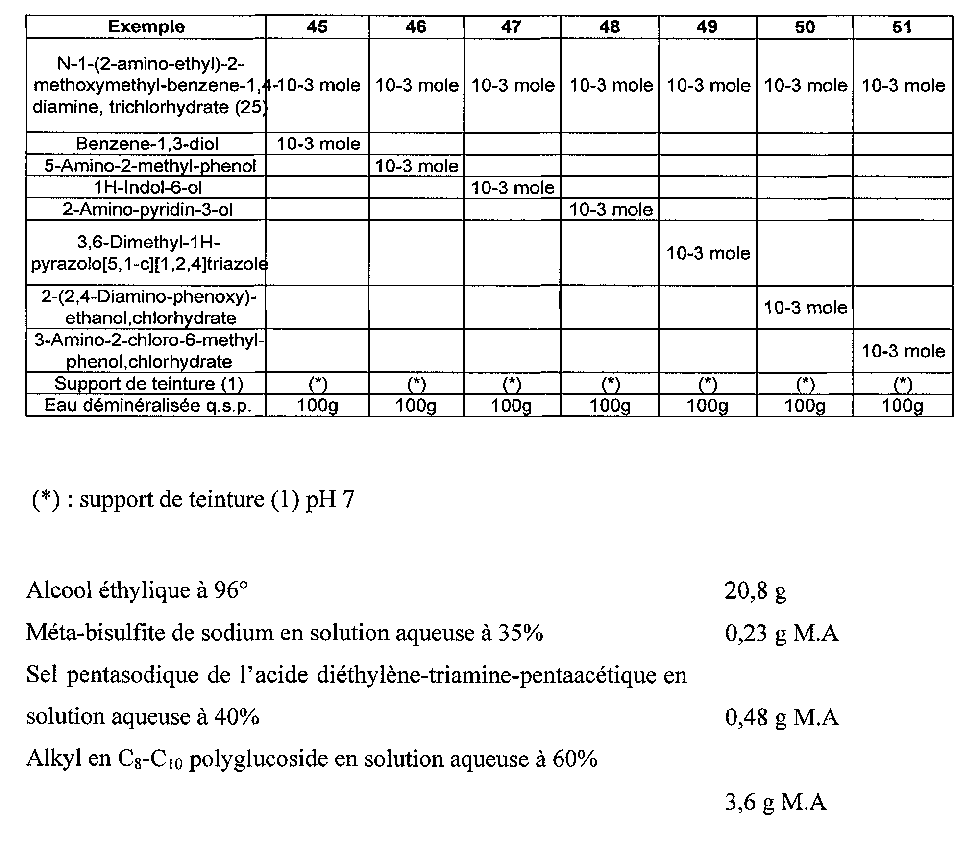

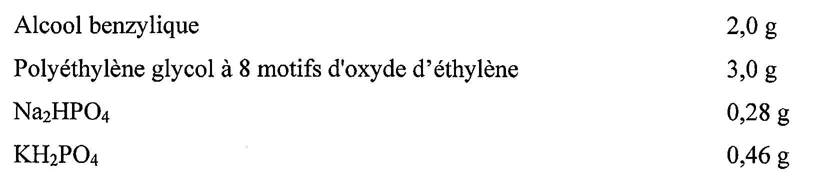

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **45** | **46** | **47** | **48** | **49** | **50** | **51** |
|---|---|---|---|---|---|---|---|
| Nuance observée | orangé | orangé | orangé intense | orangé | orangé | brun intense | orangé |

### - Exemples 52 à 55 : Teinture en milieu basique

Les compositions tinctoriales suivantes sont préparées :

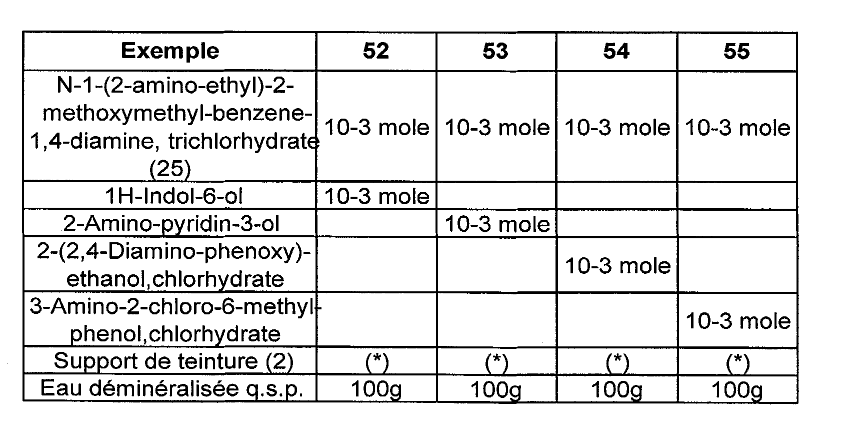

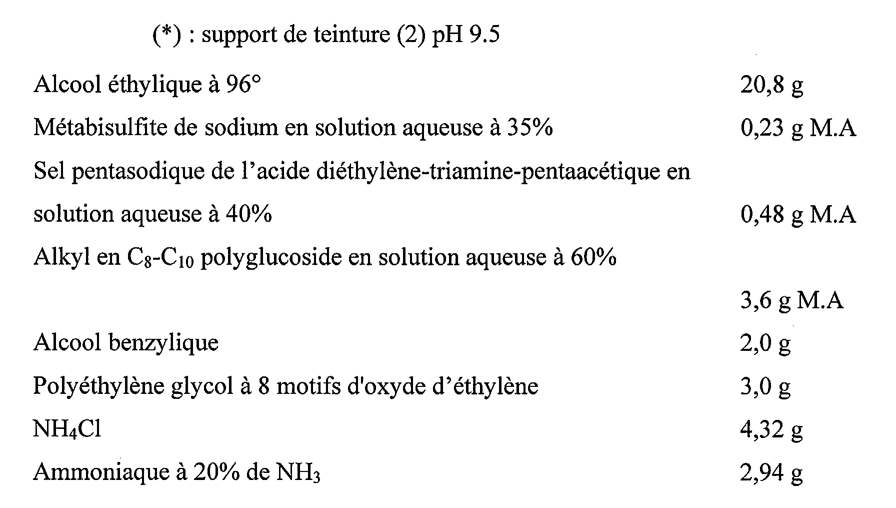

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **52** | **53** | **54** | **55** |
|---|---|---|---|---|
| **Nuance observée** | orangé | orangé | jaune | orangé |

### Exemples 63 à 71 : Composition tinctoriale à partir du 1-[3-(4-Amino-3-methyl-phenylamino)-propyl]-pyrrolidin-2-one, dichlorhydrate (27)

### - Exemples 63 à 67 : Teinture en milieu acide

Les compositions tinctoriales suivantes sont préparées :

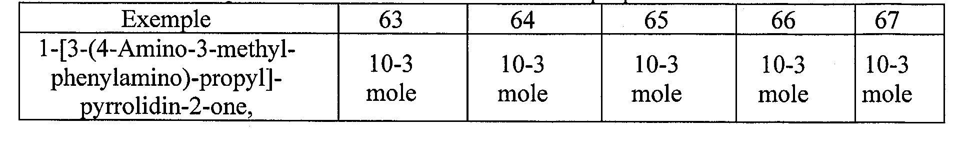

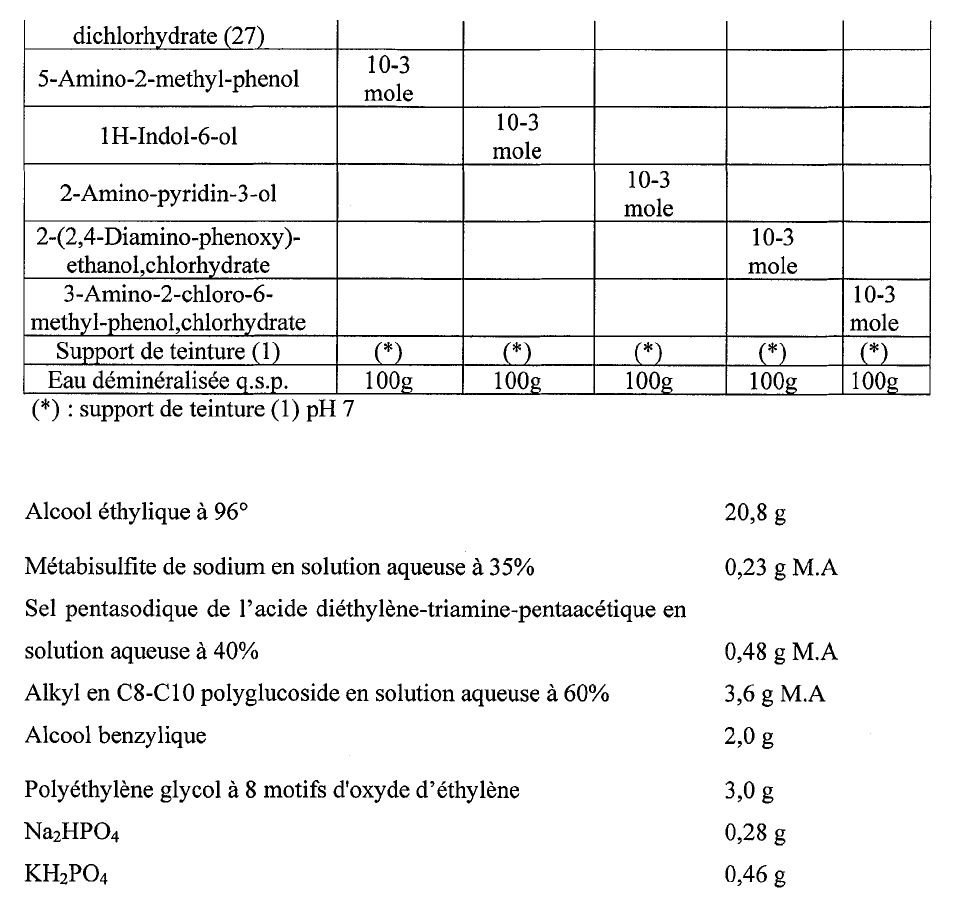

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 63 | 64 | 65 | 66 | 67 |
|---|---|---|---|---|---|
| Nuance observée | gris violet intense | orangé | brun orangé | gris vert-bleu intense | jaune |

### - Exemples 68 à 71 : teinture en milieu basique

Les compositions tinctoriales suivantes sont préparées :

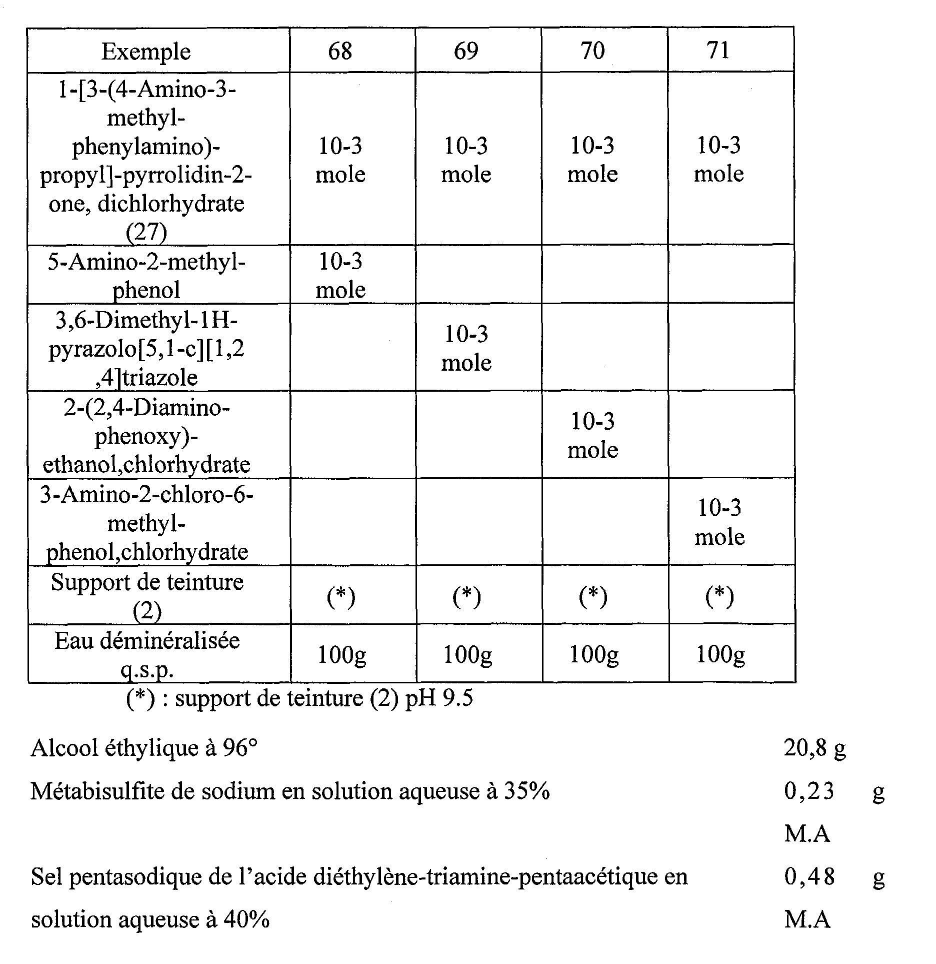

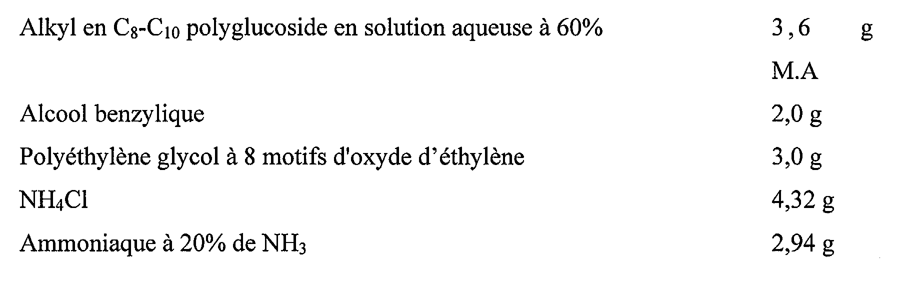

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 68 | 69 | 70 | 71 |
|---|---|---|---|---|
| Nuance observée | gris violet | rouge chromatique | vert-bleu | bleu intense |

### Exemples 72 à 83 : Composition tinctoriale à partir du du 2-Methyl-N-1-(3-pyrrolidin-1-yl-propyl)-benzene-1,4-diamine, dichlorhydrate (41)

### - Exemples 72 à 78: Teinture en milieu acide

Les compositions tinctoriales suivantes sont préparées :

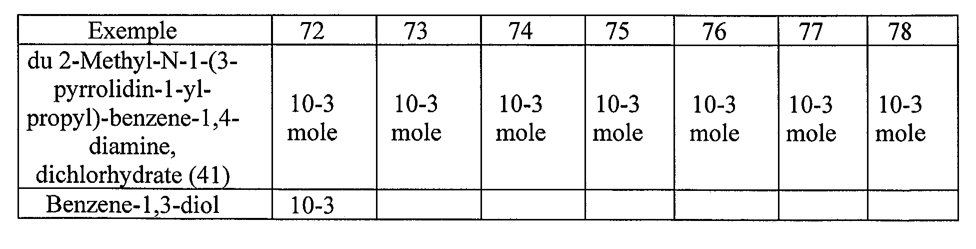

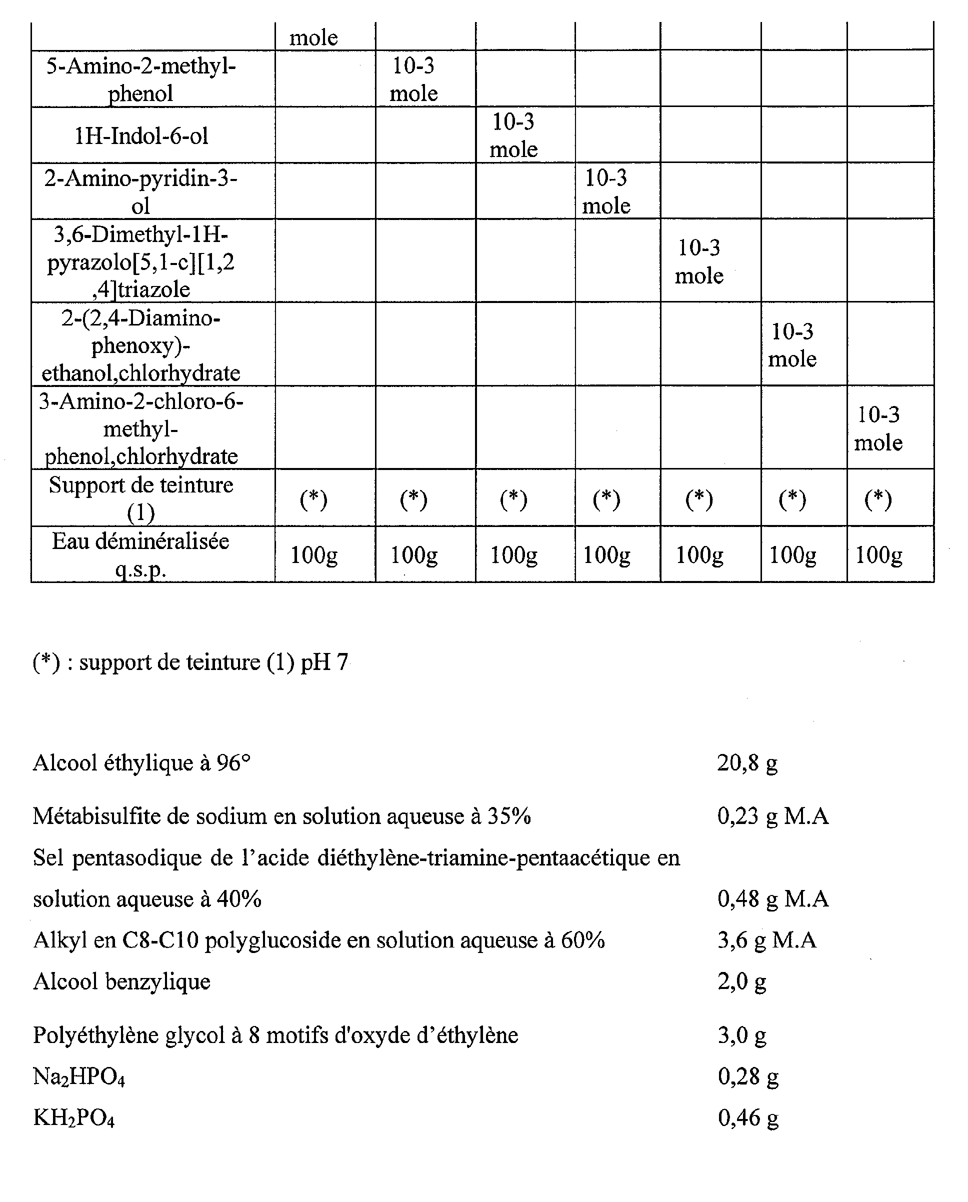

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 72 | 73 | 74 | 75 | 76 | 77 | 78 |
|---|---|---|---|---|---|---|---|
| Nuance observée | brun jaune | gris violet intense | brun | brun rouge | orangé | gris bleu intense | violet-bleu intense |

### - Exemples 79 à 83 : teinture en milieu basique

Les compositions tinctoriales suivantes sont préparées :

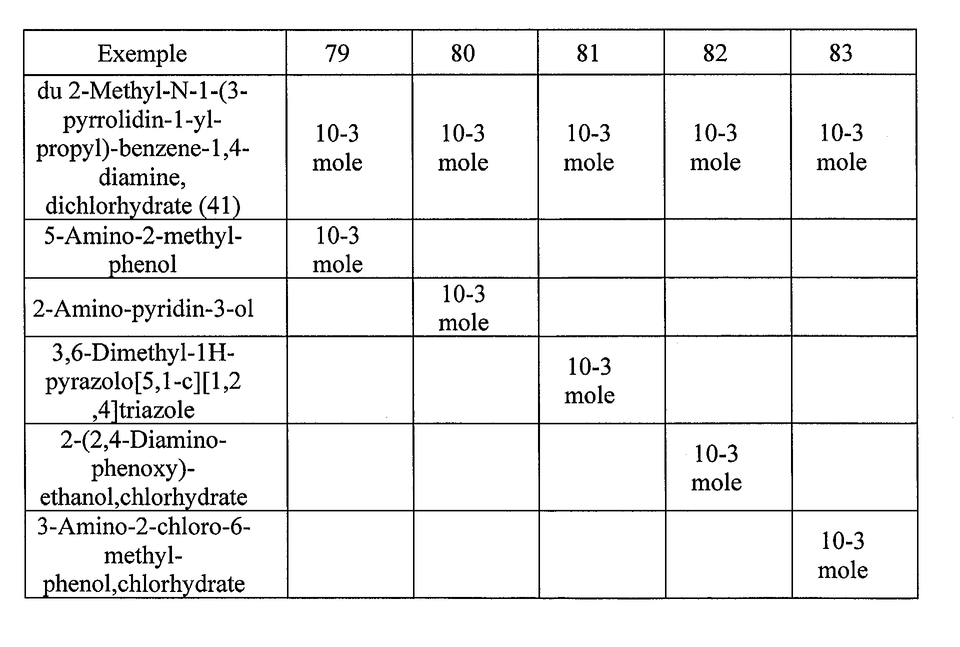

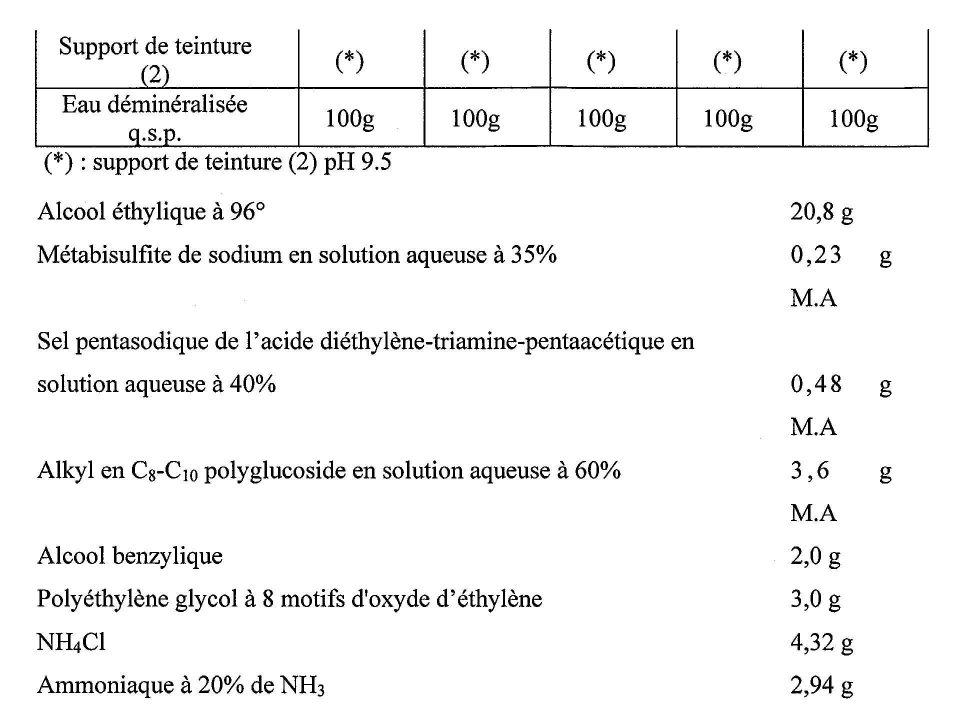

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 79 | 80 | 81 | 82 | 83 |
|---|---|---|---|---|---|
| Nuance observée | violet-rouge intense | orangé | rouge chromatique | bleu intense | violet-bleu intense |

### Exemples 84 à 92 : Composition tinctoriale à partir du N-1-(3-Diethylamino-propyl)-2-methyl-benzene-1,4-diamine, dichlorhydrate (33)

### - Exemples 84 à 88 : Teinture en milieu acide

Les compositions tinctoriales suivantes sont préparées :

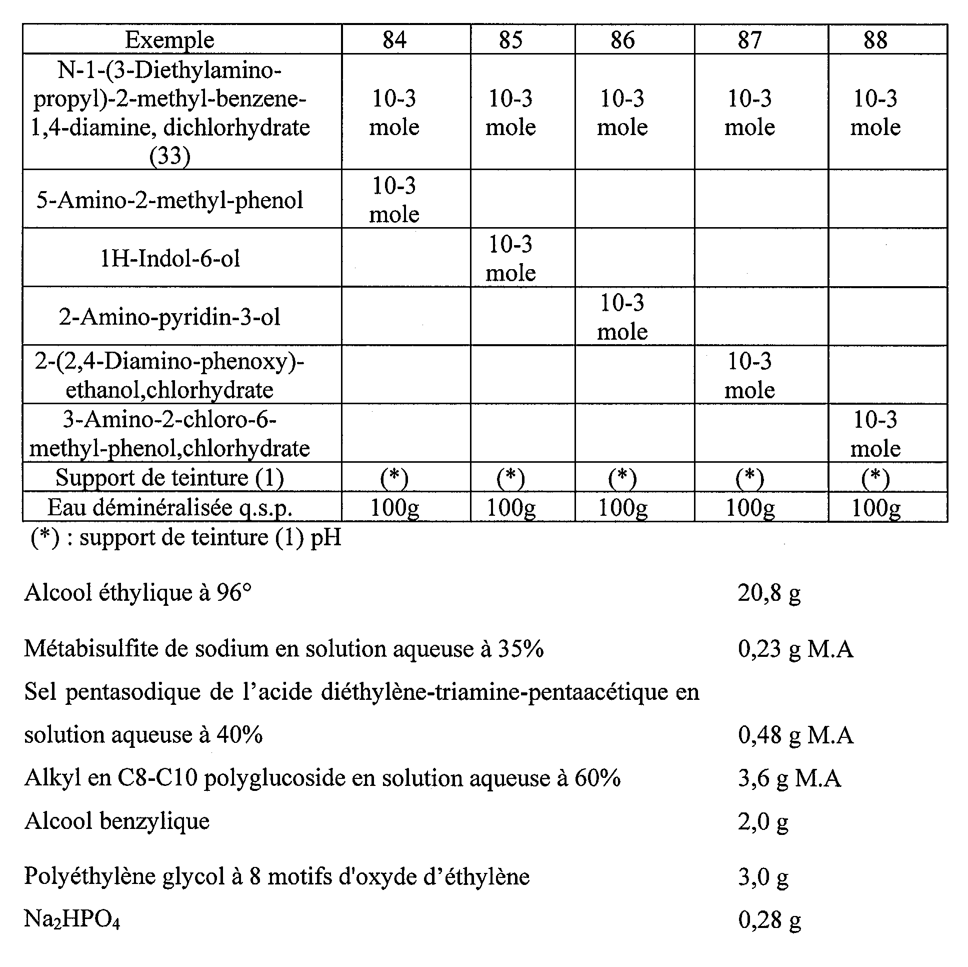

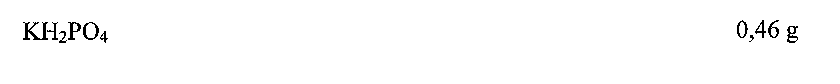

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 84 | 85 | 86 | 87 | 88 |
|---|---|---|---|---|---|
| Nuance observée | gris violet-rouge | brun | brun orangé | gris vert-bleu intense | violet-bleu intense |

### - Exemples 89 à 92 : teinture en milieu basique

Les compositions tinctoriales suivantes sont préparées :

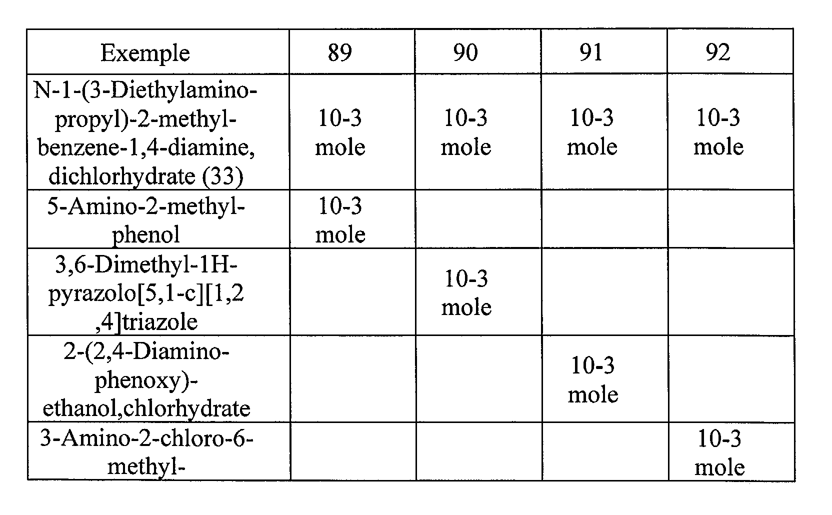

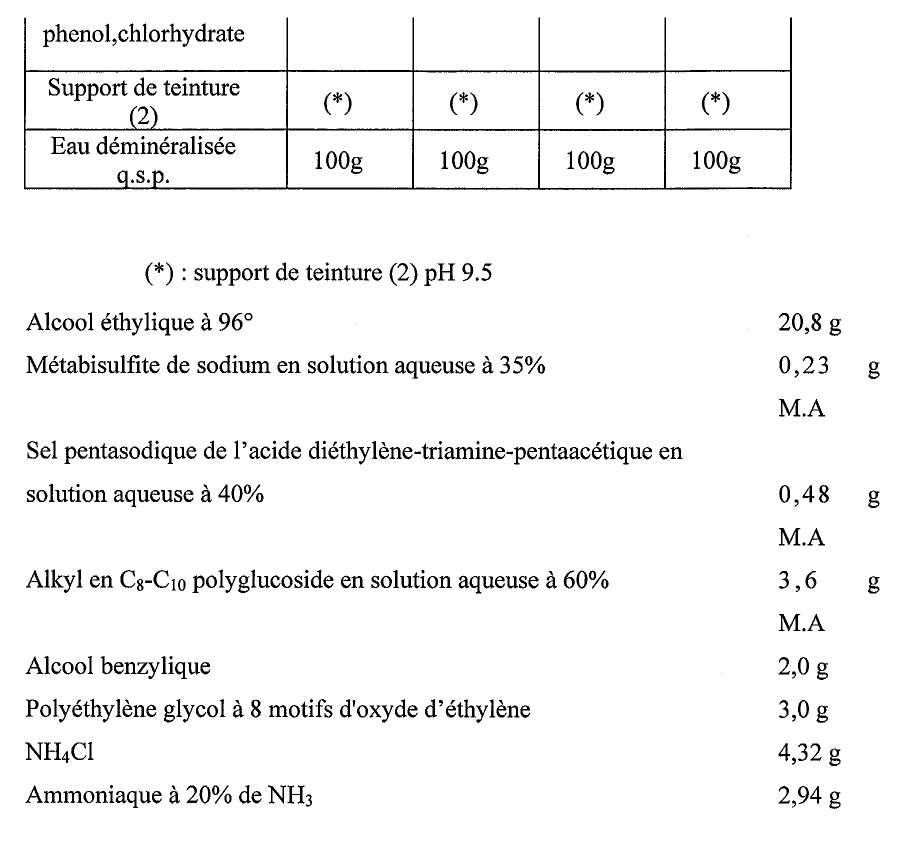

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 89 | 90 | 91 | 92 |
|---|---|---|---|---|
| Nuance observée | violet-rouge | rouge chromatique | bleu | violet-bleu intense |

### Exemples 93 à 99 : Composition tinctoriale à partir du N-1-(2-Dimethylamino-ethyl)-2-methyl-benzene-1,4-diamine, dichlorhydrate (29)

### - Exemples 93 à 95 : Teinture en milieu acide

Les compositions tinctoriales suivantes sont préparées :

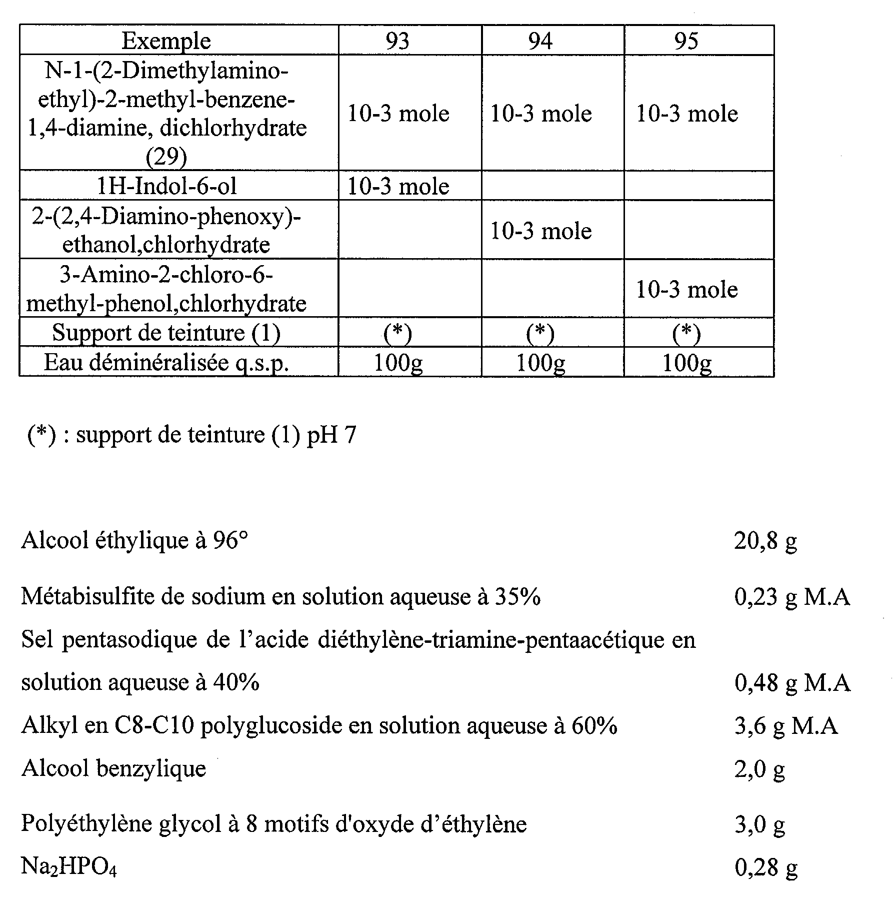

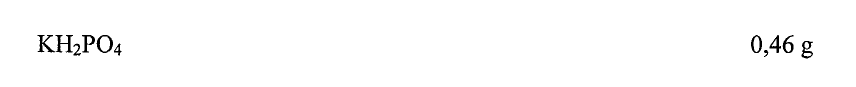

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableau ci-dessous :

| | | | |
|---|---|---|---|
| Exemple | 93 | 94 | 95 |
| Nuance observée | brun rouge | gris bleu intense | brun rouge |

### - Exemples 96 à 99 : teinture en milieu basique

Les compositions tinctoriales suivantes sont préparées :

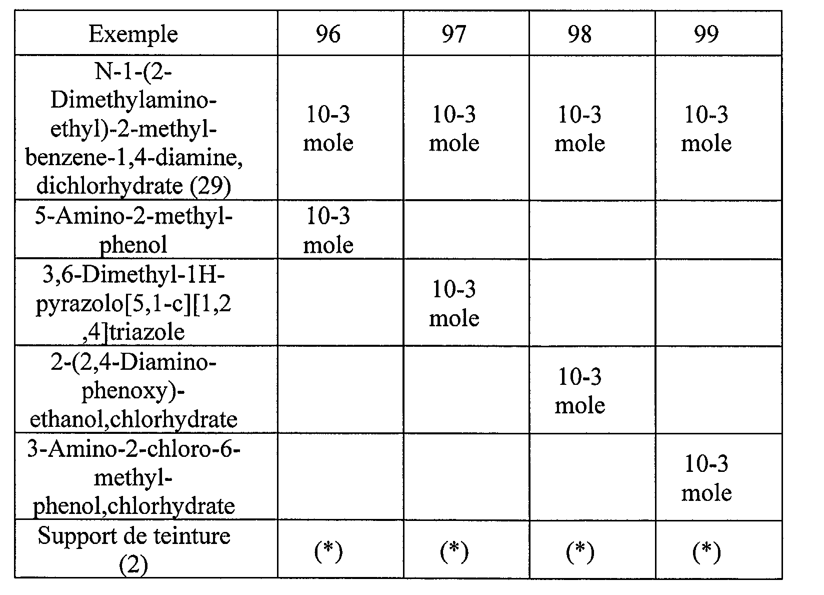

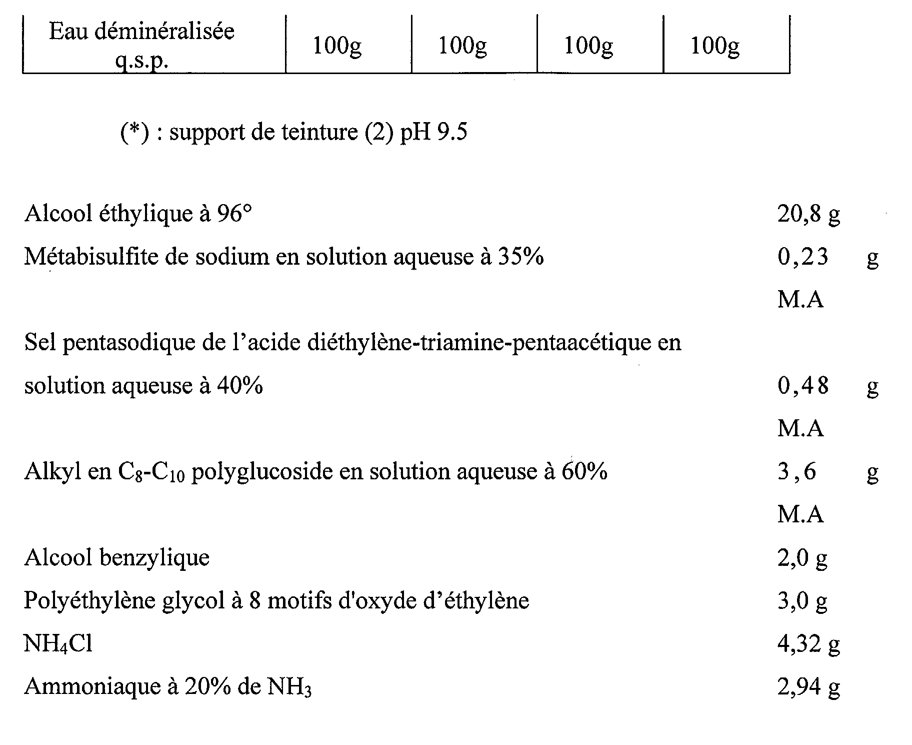

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 96 | 97 | 98 | 99 |
|---|---|---|---|---|
| Nuance observée | rouge | rouge chromatique | bleu intense | violet intense |

### Exemples 100 à 108 : Composition tinctoriale à partir du N-1-(3-dimethylamino-propyl)-2-methyl-benzene-1,4-diamine, dichlorhydrate (37)

### - Exemples 100 à 104: Teinture en milieu acide

Les compositions tinctoriales suivantes sont préparées :

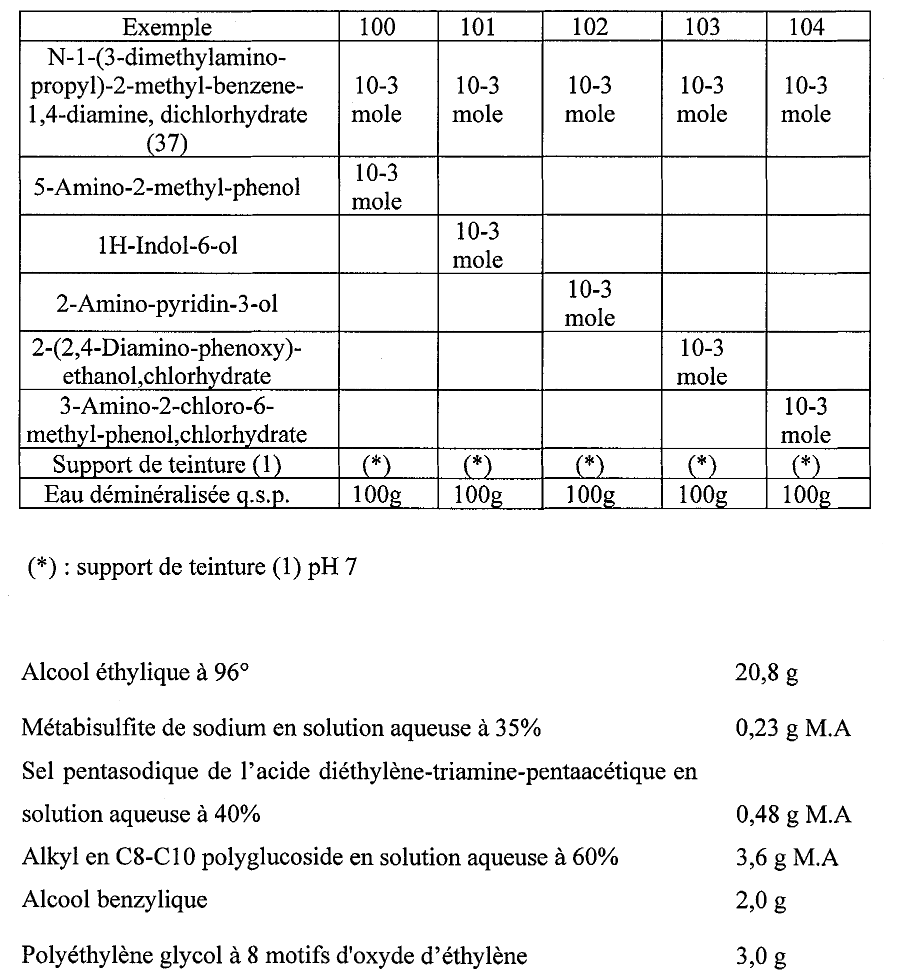

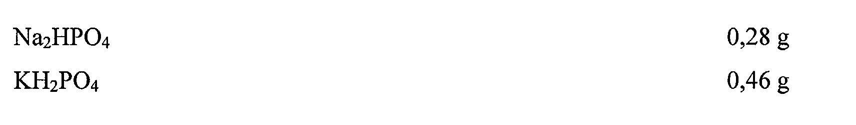

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 100 | 101 | 102 | 103 | 104 |
|---|---|---|---|---|---|
| Nuance observée | gris violet-rouge | brun | brun rouge | gris bleu intense | violet-bleu intense |

### - Exemples 105 à 108 : teinture en milieu basique

Les compositions tinctoriales suivantes sont préparées :

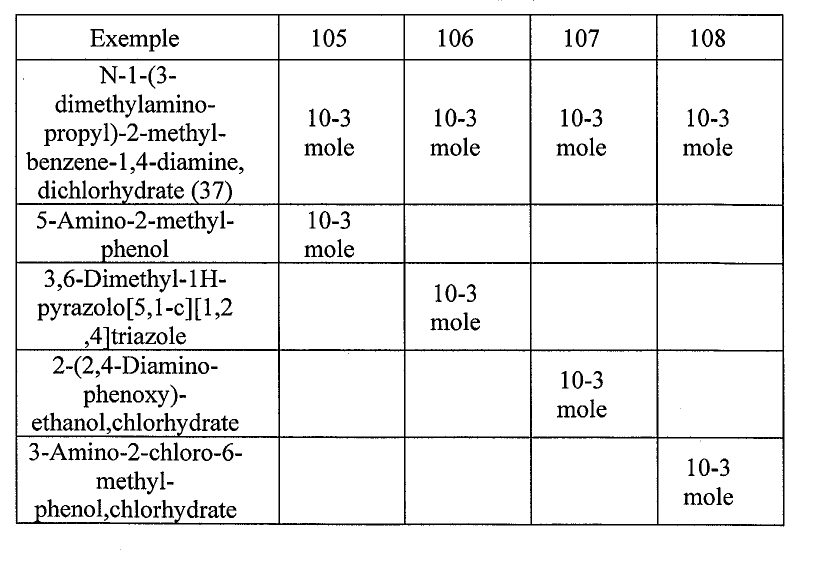

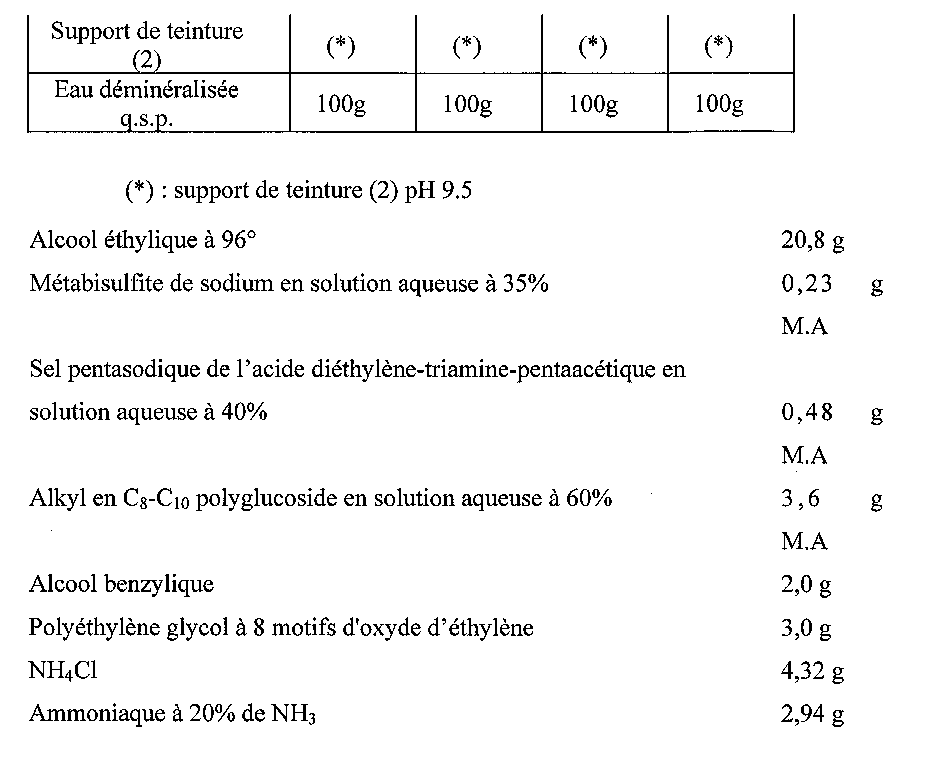

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 105 | 106 | 107 | 108 |
|---|---|---|---|---|
| Nuance observée | violet-rouge | rouge chromatique | bleu intense | violet-bleu intense |

### Exemples 109 à 122 : Composition tinctoriale à partir du N-1-(3-dibutylamino-propyl)-2-methyl-benzene-1,4-diamine, dichlorhydrate (35)

### - Exemples 109 à 115 : Teinture en milieu acide

Les compositions tinctoriales suivantes sont préparées :

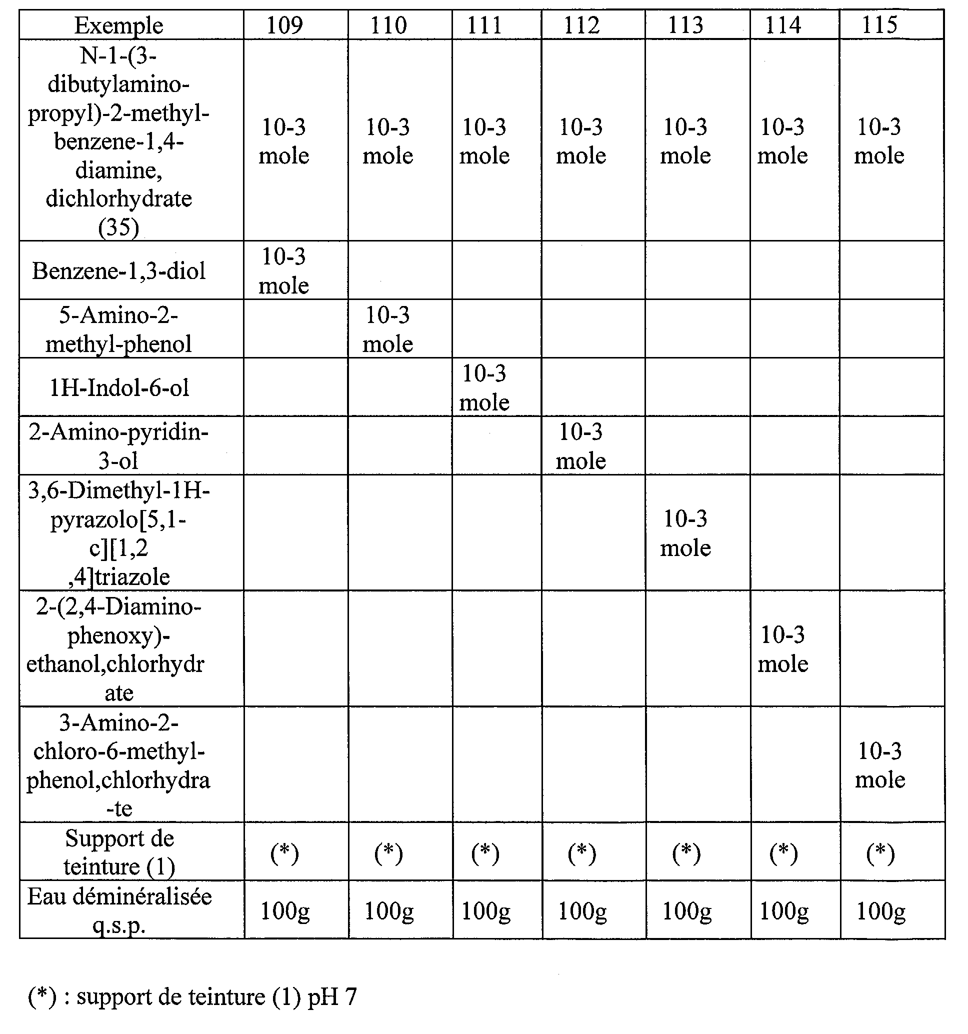

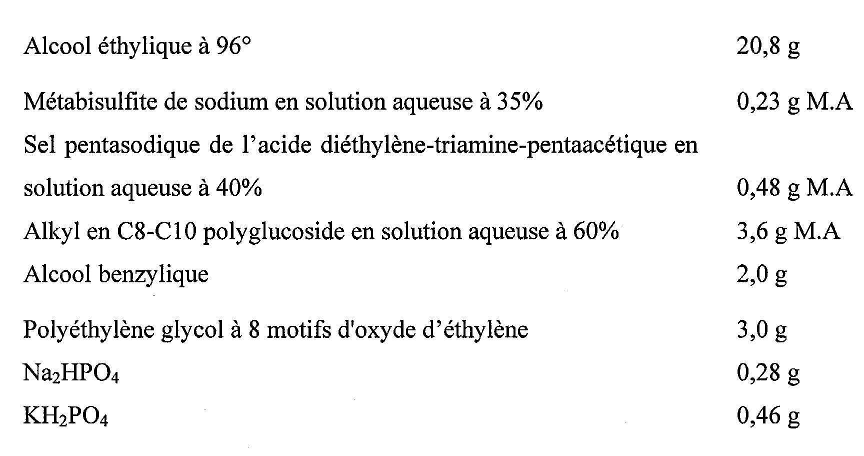

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 109 | 110 | 111 | 112 | 113 | 114 | 115 |
|---|---|---|---|---|---|---|---|
| Nuance observée | brun | violet intense | brun rouge intense | brun rouge intense | brun orangé | bleu intense | violet intense |

### - Exemples 116 à 122 : teinture en milieu basique

Les compositions tinctoriales suivantes sont préparées :

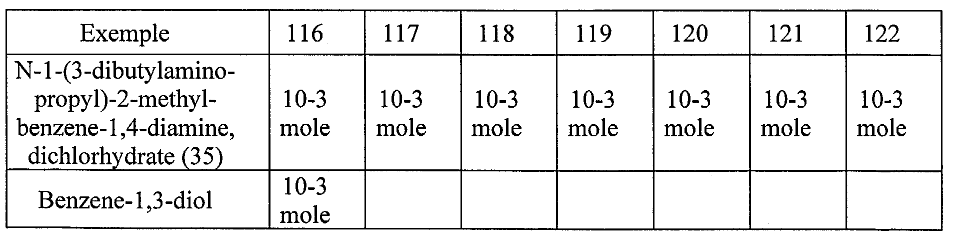

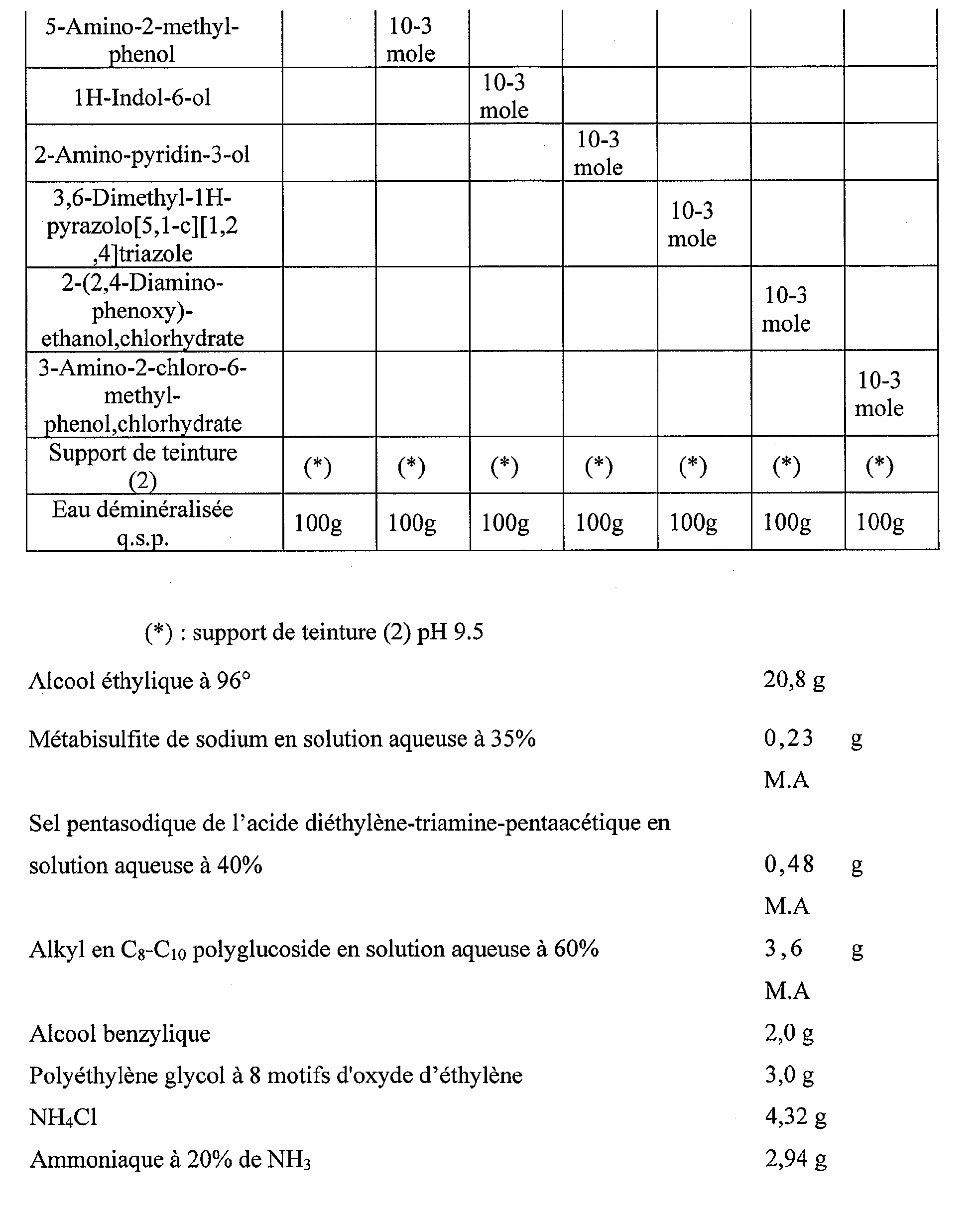

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 116 | 117 | 118 | 119 | 120 | 121 | 122 |
|---|---|---|---|---|---|---|---|
| Nuance observée | jaune | violet intense | rouge intense | brun rouge intense | rouge chroma -tique intense | bleu intense | violet intense |

### Exemples 123 à 135 : Composition tinctoriale à partir du N-1-(2-Diisopropylamino-ethyl)-2-methyl-benzene-1,4-diamine, dichlorhydrate (39)

### - Exemples 123 à 129 : Teinture en milieu acide

Les compositions tinctoriales suivantes sont préparées :

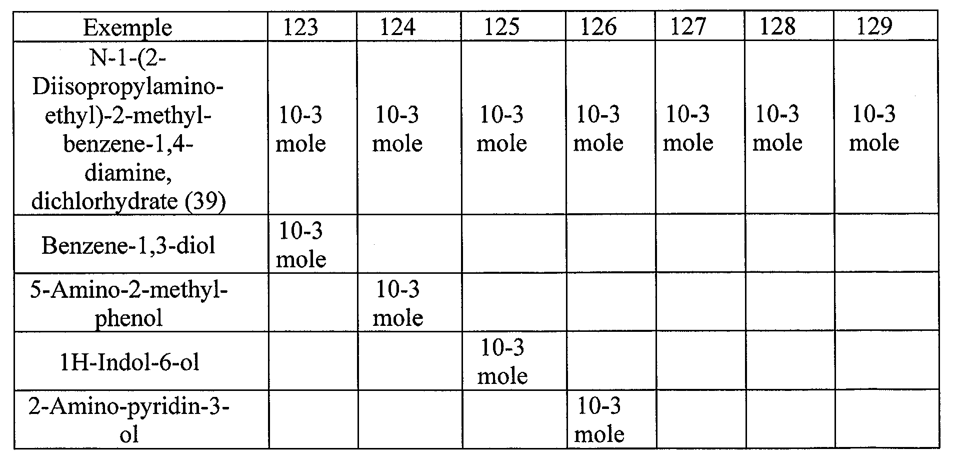

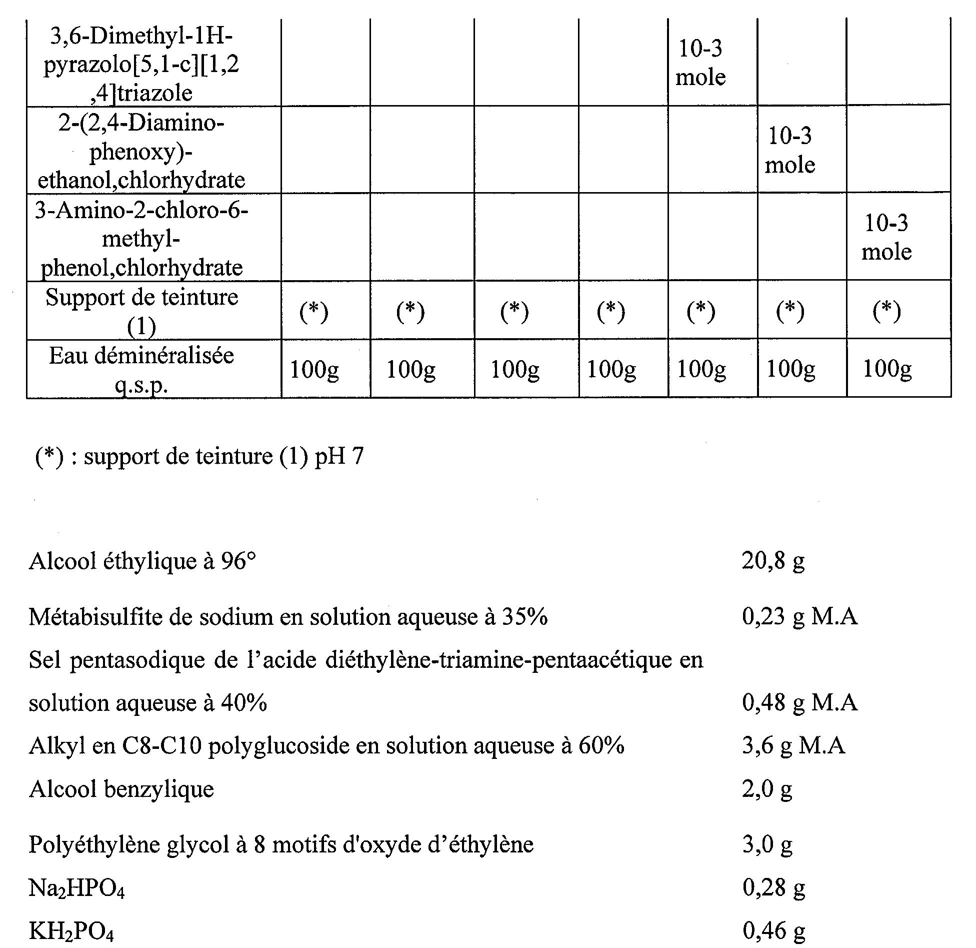

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 123 | 124 | 125 | 126 | 127 | 128 | 129 |
|---|---|---|---|---|---|---|---|
| Nuance observée | orangé | violet-rouge intense | brun intense | rouge intense | orangé | gris bleu intense | gris violet-rouge intense |

### - Exemples 130 à 135: teinture en milieu basique

Les compositions tinctoriales suivantes sont préparées :

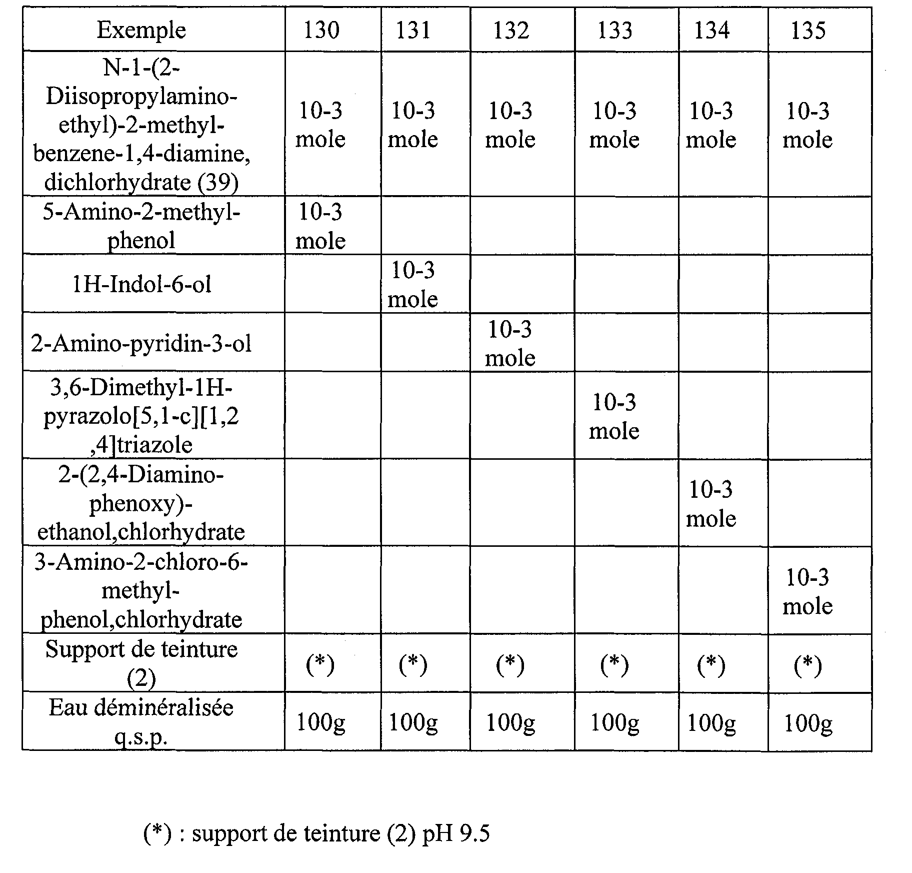

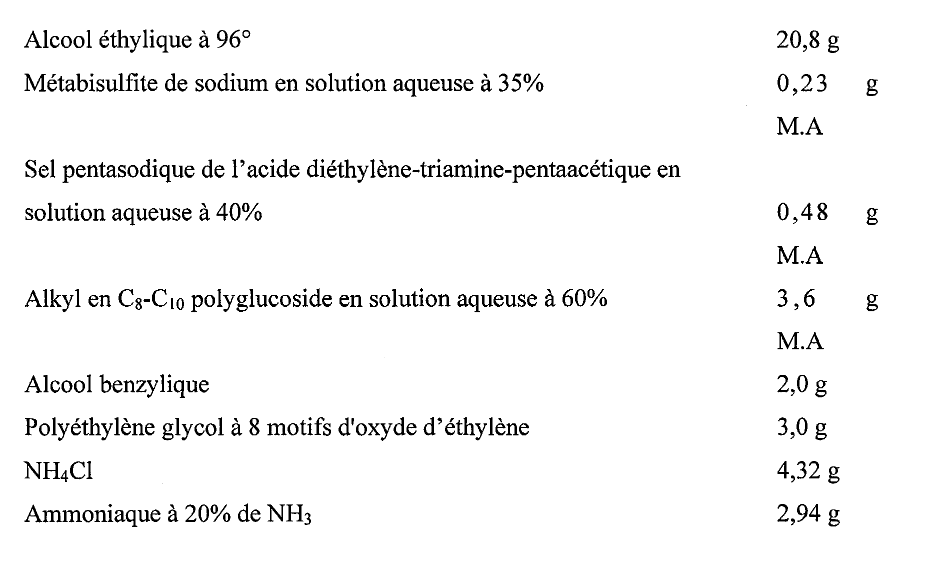

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 130 | 131 | 132 | 133 | 134 | 135 |
|---|---|---|---|---|---|---|
| Nuance observée | rouge | orangé | orangé | rouge chromatique | bleu intense | violet intense |

### Exemples 136 à 141 : Composition tinctoriale à partir du 2-Methyl-N-4-(2-pyrrolidin-1-yl-ethyl)-benzene-1,4-diamine, dichlorhydrate (31)

### - Exemples 136 à 138 : Teinture en milieu acide

Les compositions tinctoriales suivantes sont préparées :

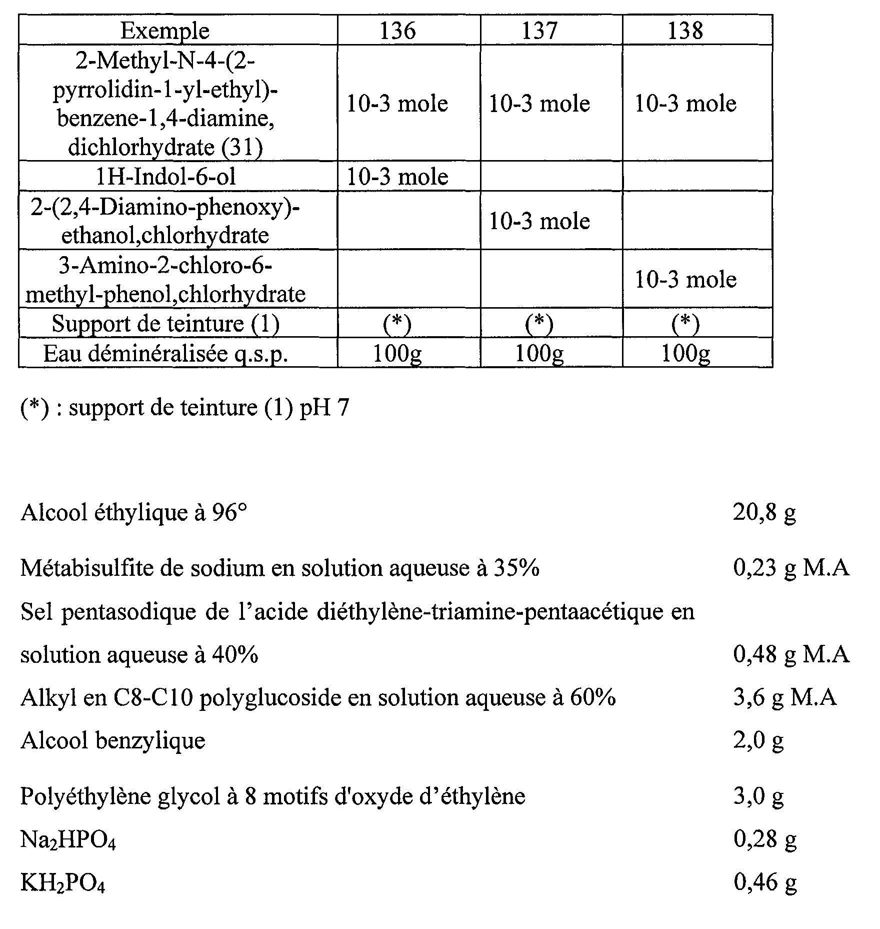

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 136 | 137 | 138 |
|---|---|---|---|
| Nuance observée | orangé | gris bleu intense | gris violet-rouge |

### - Exemples 139 à 141 : teinture en milieu basique

Les compositions tinctoriales suivantes sont préparées :

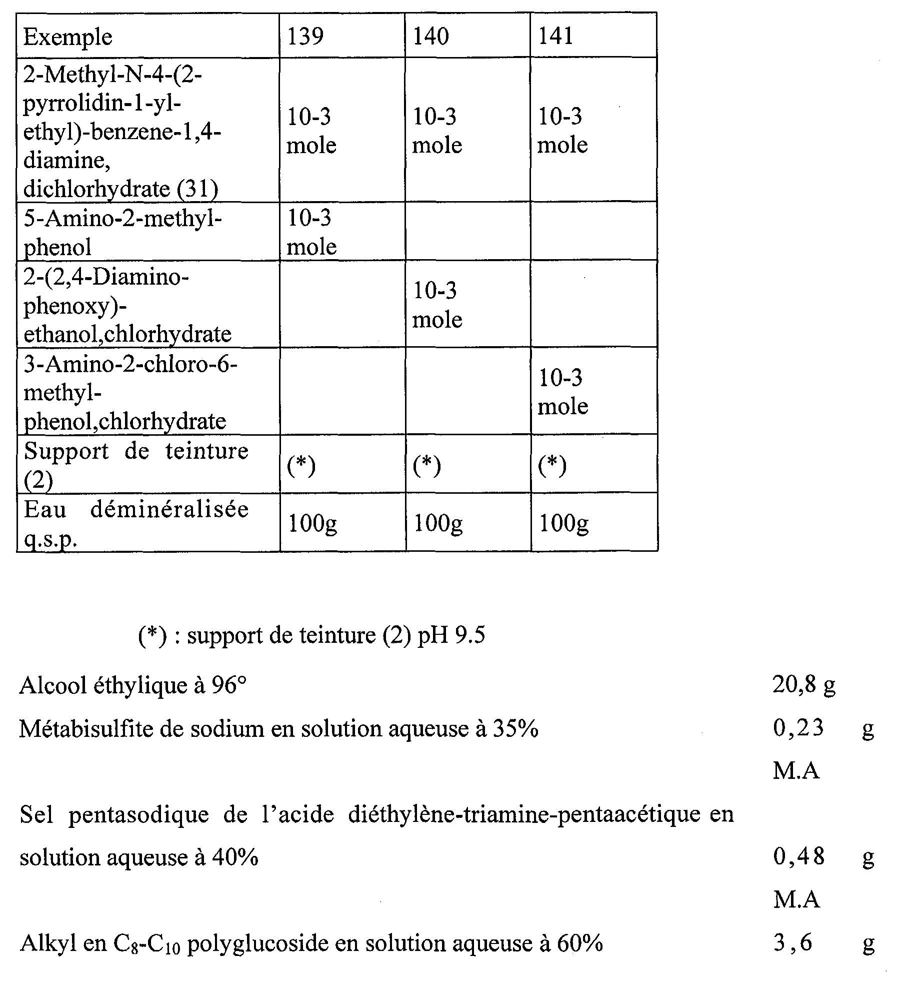

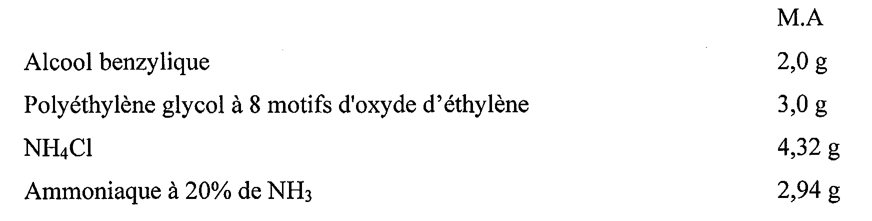

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 139 | 140 | 141 |
|---|---|---|---|
| Nuance observée | rouge | gris bleu | violet intense |

### Exemples 142 à 151 : Composition tinctoriale à partir du 1-[3-(4-Amino-2-methyl-phenylamino)-propyl]-pyrrolidin-2-one, dichlorhydrate (45)

### - Exemples 142 à 147 : Teinture en milieu acide

Les compositions tinctoriales suivantes sont préparées :

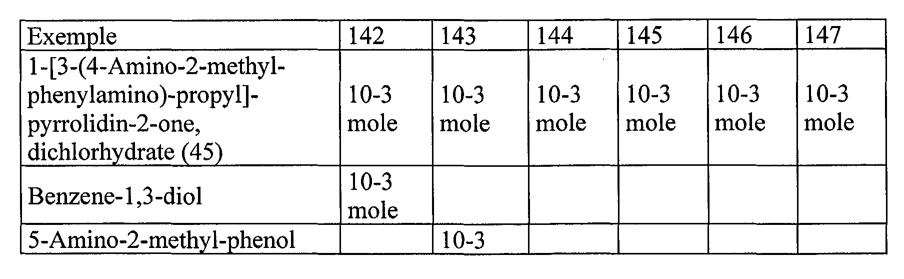

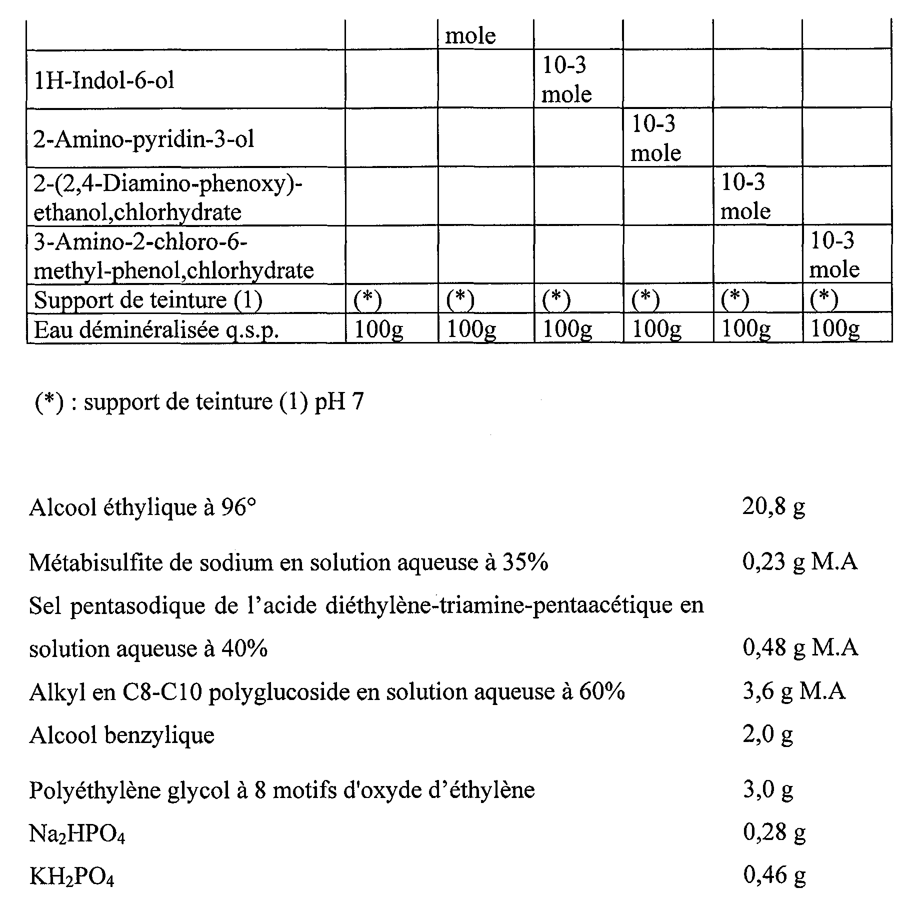

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 142 | 143 | 144 | 145 | 146 | 147 |
|---|---|---|---|---|---|---|
| Nuance observée | brun jaune | gris violet intense | brun brun intense | brun | vert-bleu intense | violet-bleu intense |

### - Exemples 148 à 151 : teinture en milieu basique

Les compositions tinctoriales suivantes sont préparées :

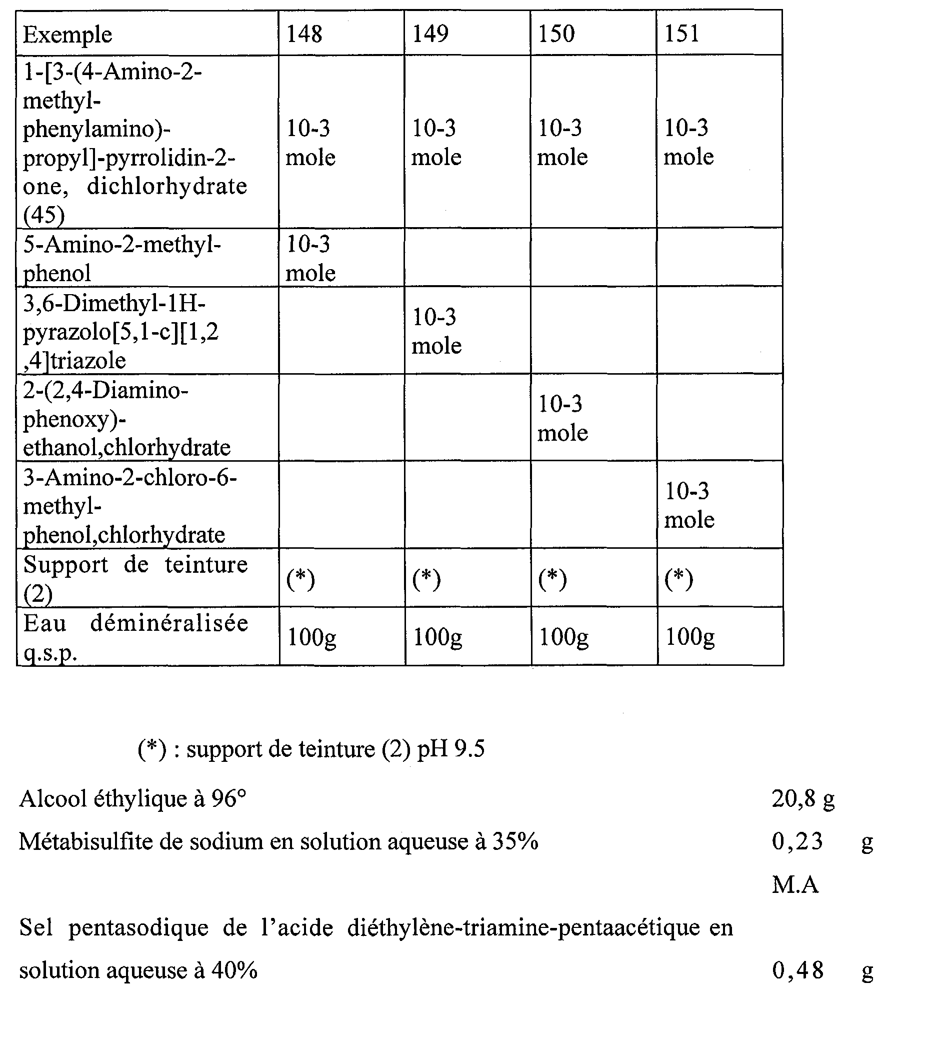

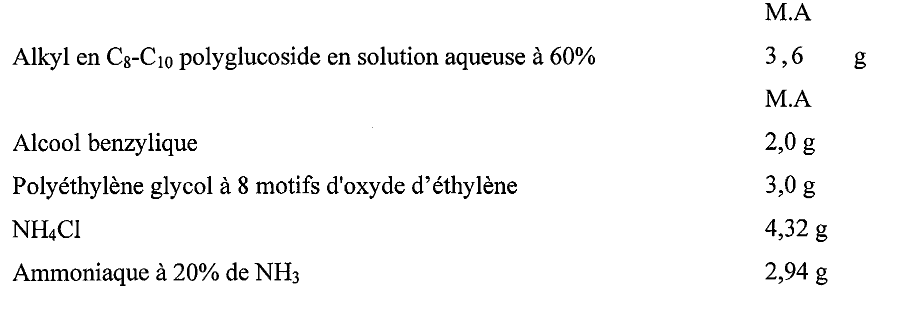

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 148 | 149 | 150 | 151 |
|---|---|---|---|---|
| Nuance observée | violet | rouge chromatique | bleu intense | violet-bleu intense |

### Exemples 152 à 158 : Composition tinctoriale à partir du 3-Methyl-N-4-(2-pyrrolidin-1-yl-ethyl)-benzene-1,4-diamine, dichlorhydrate (47)

### - Exemples 152 à 154 : Teinture en milieu acide

Les compositions tinctoriales suivantes sont préparées :

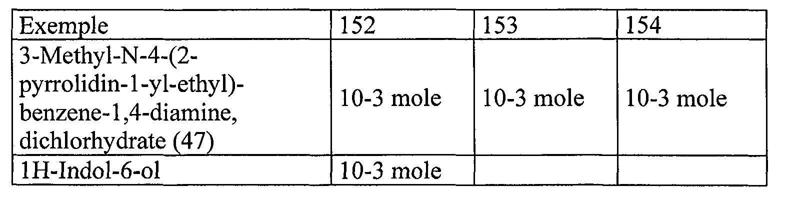

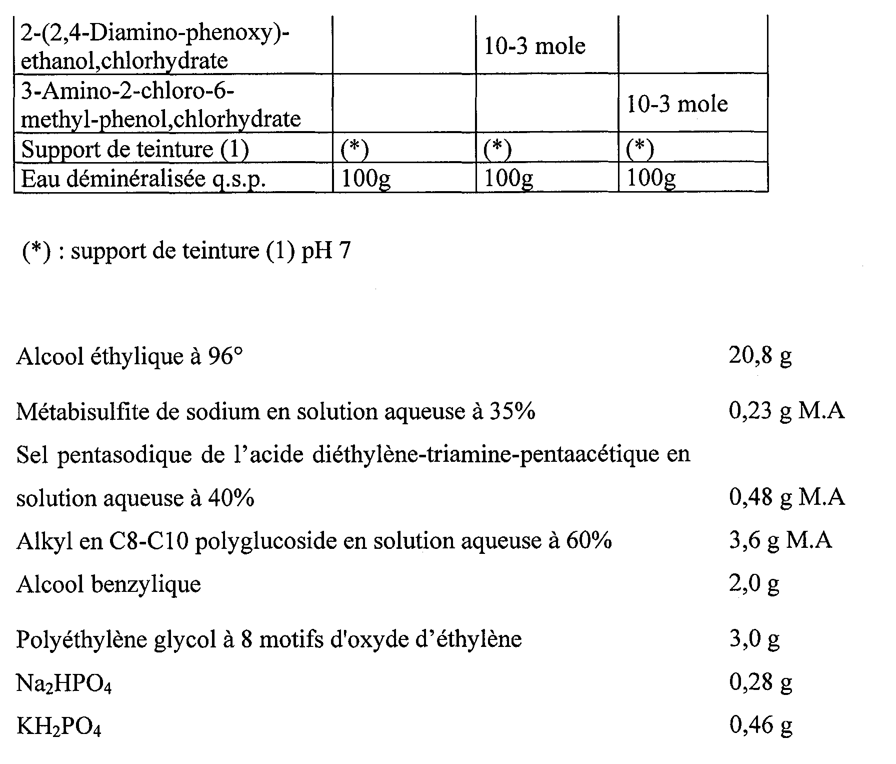

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 152 | 153 | 154 |
|---|---|---|---|
| Nuance observée | brun rouge | gris bleu intense | gris violet- rouge |

### - Exemples 155 à 158 : teinture en milieu basique

Les compositions tinctoriales suivantes sont préparées :

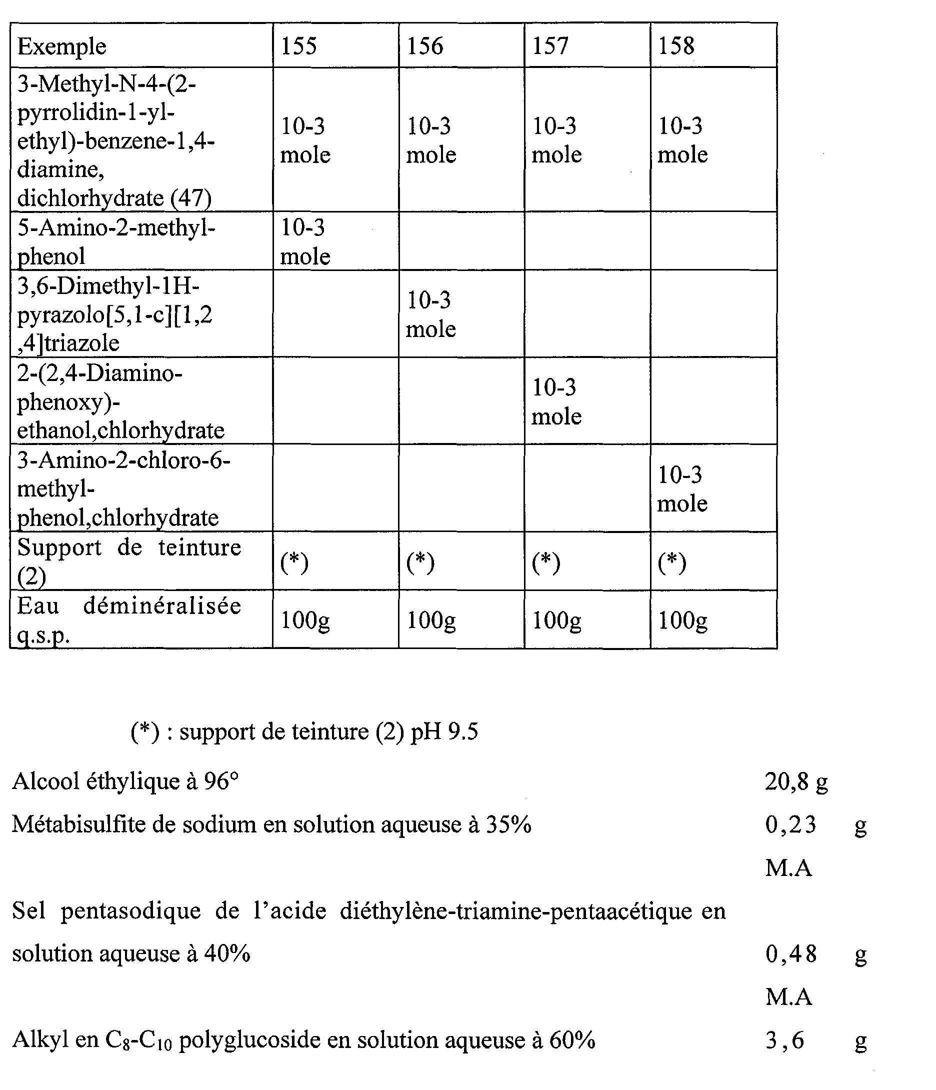

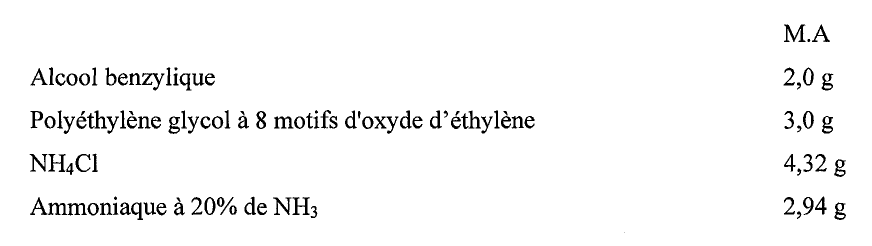

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 155 | 156 | 157 | 158 |
|---|---|---|---|---|
| Nuance observée | rouge | rouge chromatique | bleu intense | violet intense |

### Exemples 159 à 167 : Composition tinctoriale à partir du 3-Methyl-N-1-(3-pyrrolidin-1-yl-propyl)-benzene-1,4-diamine, dichlorhydrate (43)

### - Exemples 159 à 163 : Teinture en milieu acide

Les compositions tinctoriales suivantes sont préparées :

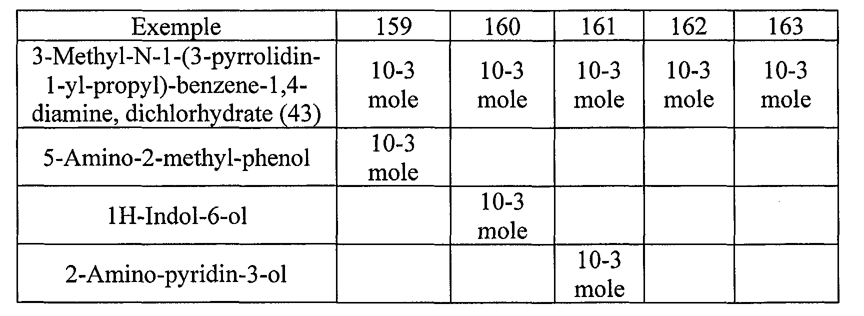

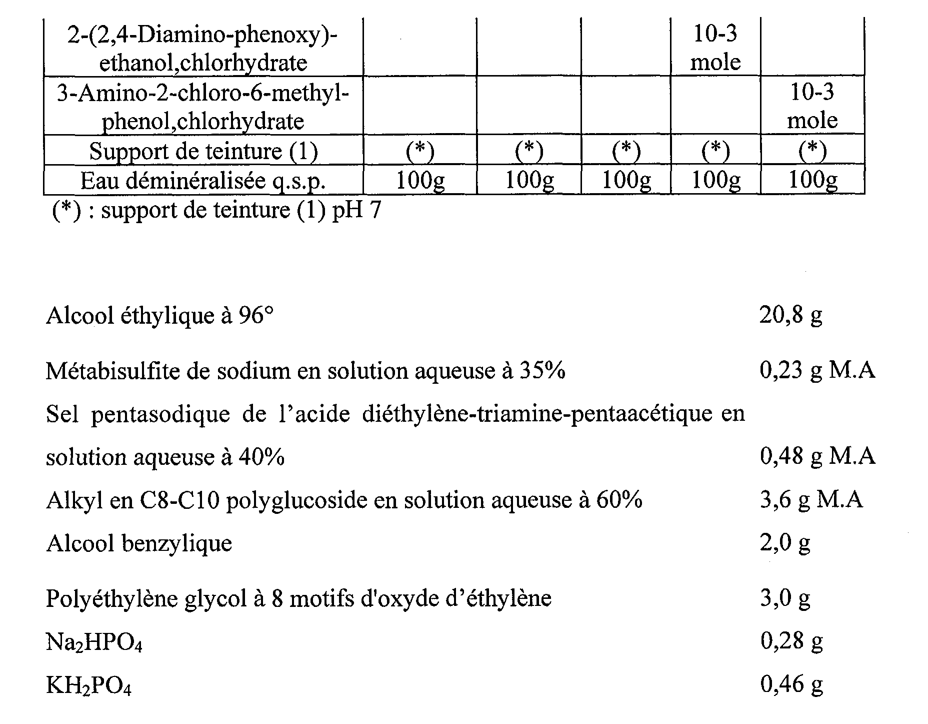

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 159 | 160 | 161 | 162 | 163 |
|---|---|---|---|---|---|
| Nuance observée | gris violet-rouge | orangé | brun orangé | gris vert-bleu intense | violet-bleu intense |

### - Exemples 164 à 167 : teinture en milieu basique

Les compositions tinctoriales suivantes sont préparées :

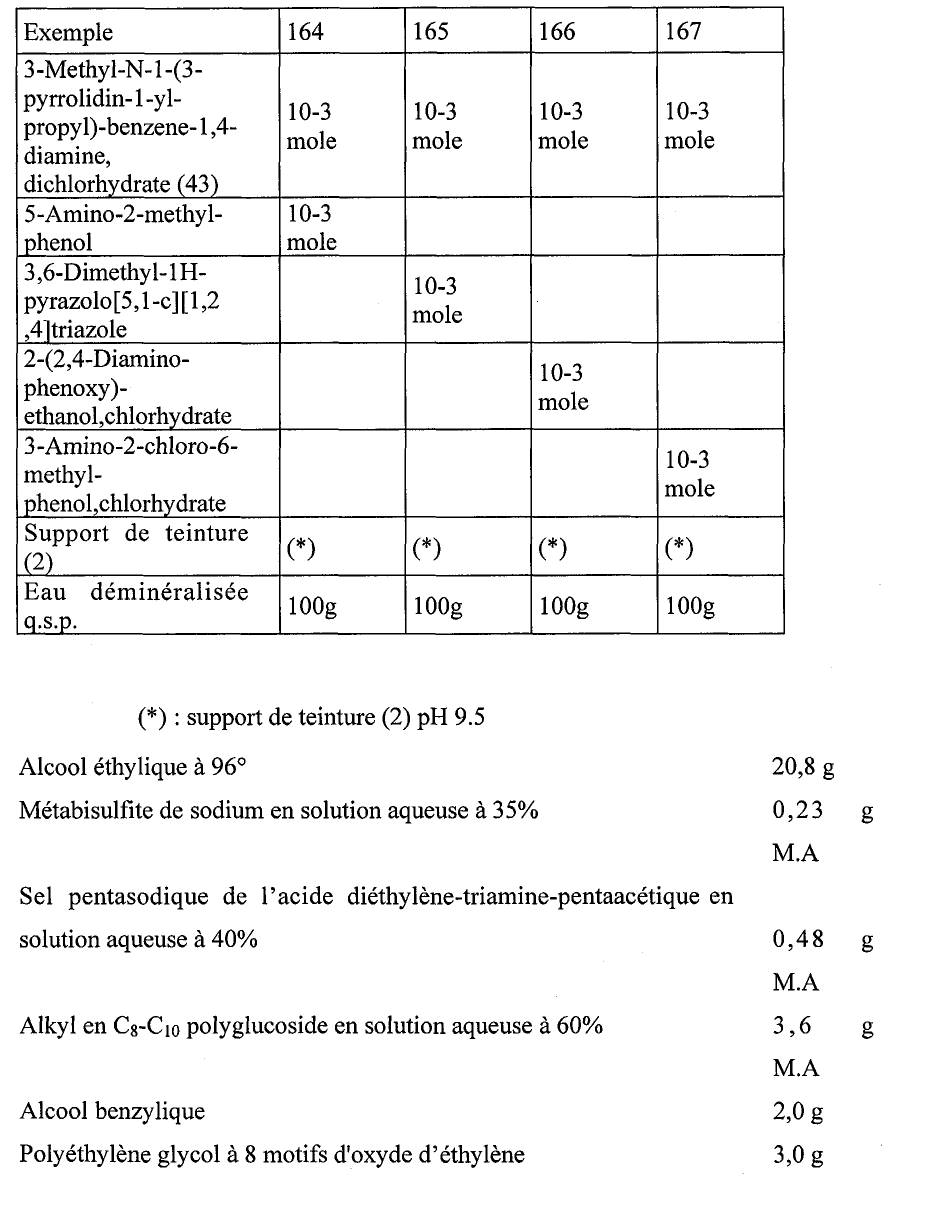

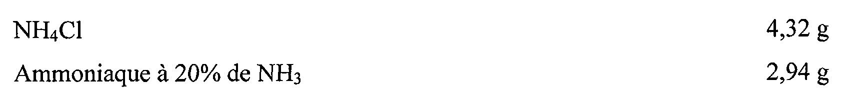

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous

| Exemple | 164 | 165 | 166 | 167 |
|---|---|---|---|---|
| Nuance observée | violet | rouge chroma-tique | vert-bleu | violet-bleu intense |

## Revendications

1. Composé **caractérisé par le fait qu'**il s'agit d'une para-phénylènediamine secondaire N-alkylaminée ortho-substituée et/ou méta-substituée de formule générale (I) : 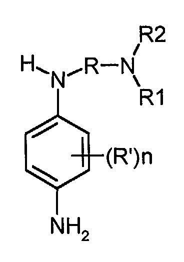 dans laquelle :
les radicaux R₁ et R₂ représentent, indépendamment l'un de l'autre, un atome d'hydrogène, un groupement alkyle, ou les radicaux R₁ et R₂ forment, ensemble et avec l'azote qu'ils substituent, un cycle saturé à 5 ou 6 chaînons éventuellement interrompu par un ou plusieurs atomes d'azote et/ou par un ou plusieurs groupements carbonyle, et éventuellement substitué par un ou plusieurs groupements alkyles,
le radical R représente un radical alkylène en C₂-C₁₀, linéaire ou ramifié, éventuellement interrompu par un ou plusieurs atomes choisis parmi l'azote ou l'oxygène et éventuellement substitué par un ou plusieurs groupements choisis parmi les groupements amino, monoalkylamino, dialkylamino, alkylcarbonyle, amido, alkoxycarbonyle, monoalkylaminocarbonyle, dialkylaminocarbonyle,
le radical R' représente un radical choisi parmi les radicaux alkyle, alcoxy, alcoxyalkyle, monohydroxyalkyle, polyhydroxyalkyle et l'atome de chlore,
n est un entier égal à 1,
sous réserve que le composé de formule (I) ne soit pas un des composés suivants : N-1-(2-Diethylamino-ethyl)-2-methoxy-benzene-1,4-diamine ; N-1-(3-Diethylamino-propyl)-3-methyl-benzene-1,4-diamine et le N-1-(3 pyrrolidino-propyl)-3-methyl-benzene-1,4 diamine.

2. Composé de formule (I) selon la revendication 1 dans laquelle R1 représente un atome d'hydrogène ou un groupement alkyle, R2 étant tel que défini à la revendication 1.

3. Composé de formule (I) selon la revendication 2 dans laquelle R₁ représente un atome d'hydrogène et le radical R₂ représente un groupement choisi parmi l'hydrogène ou un groupement alkyle.

4. Composé de formule (I) selon la revendication 1 ou 2 dans laquelle R₁ et R₂ représentent tous les deux des groupements alkyles.

5. Composé de formule (I) selon la revendication 4 dans laquelle R₁ et R₂ représentent deux groupements alkyles identiques.

6. Composé de formule (I) selon la revendication 1 dans laquelle R₁ et R₂ forment ensemble avec l'azote qu'ils substituent un cycle saturé à 5 ou 6 chaînons éventuellement interrompu par un ou plusieurs atomes d'azote et/ou un ou plusieurs groupements carbonyles et éventuellement substitué par un ou plusieurs groupements alkyles.

7. Composé de formule (I) selon la revendication 6 dans laquelle R₁ et R₂ forment ensemble avec l'azote qu'ils substituent un cycle saturé à 5 ou 6 chaînons éventuellement interrompu par un atome d'azote ou un groupement carbonyle ou éventuellement substitué par un groupement alkyle en C₁-C₃

8. Composé de formule (I) selon l'une des revendications précédentes dans laquelle R représente un radical alkylène linéaire ou ramifié en C₂-C₈, éventuellement interrompu par un ou deux atomes d'azote.

9. Composé de formule (I) selon l'une des revendications dans laquelle R' est un groupement choisi parmi les groupements alkyle en C₁-C₃, alcoxy en C₁-C₃, alcoxy en (C₁-C₃)-alkyle en (C₁-C₃) et l'atome de chlore.

10. Composé de formule (I) selon l'une des revendications précédentes **caractérisé par le fait qu'**il est choisi parmi: N-1-(2-Amino-ethyl)-2-methoxymethyl-benzene-1,4-diamine, 1-[3-(4-Amino-2-methyl-phenylamino)-propyl]-pyrrolidin-2-one ; 2-Methyl-N-1-[3-(2-methyl-piperidin-1-yl)-propyl]-benzene-1,4-diamine ; N-1-(4-Diethylamino-1-methyl-butyl)-2-methyl-benzene-1,4-diamine ; N-1-(3-Dibutylamino-propyl)-2-methyl-benzene-1,4-diamine ; N-1-(2-Dimethylamino-1-methyl-ethyl)-2-methyl-benzene-1,4-diamine ; N-1-(2-Diisopropylamino-ethyl)-2-methyl-benzene-1,4-diamine ; N-1-(2-Dibutylamino-ethyl)-2-methyl-benzene-1,4-diamine ; 2-Methyl-N-1-(3-pyrrolidin-1-yl-propyl)-benzene-1,4-diamine ; 2-Methyl-N-1-(2-pyrrolidin-1-yl-ethyl)-benzene-1,4-diamine ; N-1-(4-Amino-butyl)-2-methyl-benzene-1,4-diamine ; N-1-(2-Isopropylamino-ethyl)-2-methyl-benzene-1,4-diamine ; N-1-(2-Dimethylamino-ethyl)-2-methyl-benzene-1,4-diamine ; N-1-(3-Dimethylamino-propyl)-2-methyl-benzene-1,4-diamine ; N-1-(8-Amino-octyl)-2-methyl-benzene-1,4-diamine ; N-1-(6-Amino-hexyl)-2-methyl-benzene-1,4-diamine ; N-1-{2-[2-(2-Dimethylamino-ethoxy)-ethoxy]-ethyl}-2-methyl-benzene-1, 4-diamine ; 2-Methyl-N-1-[3-(4-methyl-piperazin-1-yl)-propyl]-benzene-1,4-diamine ; 1-[3-(4-Amino-3-methyl-phenylamino)-propyl]-pyrrolidin-2-one ; N-1-(3-Diethylamino-propyl)-2-methyl-benzene-1,4-diamine ; N-1-(3-Dimethylamino-2,2-dimethyl-propyl)-2-methyl-benzene-1,4-diamine ; 2-Methyl-N-1-(2-piperidin-1-yl-ethyl)-benzene-1,4-diamine ; 3-Methyl-N-1-[3-(2-methyl-piperidin-1-yl)-propyl]-benzene-1,4-diamine ; N-1-(4-Diethylamino-1-methyl-butyl)-3 -methyl-benzene-1,4-diamine ; N-1-(3-Dibutylamino-propyl)-2-methyl-benzene-1,4-diamine ; N-1-(2-Dimethylamino-1-methyl-ethyl)-3-methyl-benzene-1,4-diamine ; N-1-(2-Diisopropylamino-ethyl)-3-methyl-benzene-1,4-diamine ; N-1-(2-Dibutylamino-ethyl)-3-methyl-benzene-1,4-diamine ; 3-Methyl-N-1-(3-pyrrolidin-1-yl-propyl)-benzene-1,4-diamine ; 3-Methyl-N-1-(2-pyrrolidin-1-yl-ethyl)-benzene-1,4-diamine ; N-1-(4-Amino-butyl)-3-methyl-benzene-1,4-diamine ; N-1-(2-Isopropylamino-ethyl)-3-methyl-benzene-1,4-diamine ; N-1-(2-Dimethylamino-ethyl)-3-methyl-benzene-1,4-diamine ; N-1-(3-Dimethylamino-propyl)-3-methyl-benzene-1,4-diamine ; N-1-(8-Amino-octyl)-3-methyl-benzene-1,4-diamine ; N-1-(6-Amino-hexyl)-3-methyl-benzene-1,4-diamine ; N-1-{2-[2-(2-Dimethylamino-ethoxy)-ethoxy]-ethyl}-3-methyl-benzene-1,4-diamine ; 3-Methyl-N-1-[3-(4-methyl-piperazin-1-yl)-propyl]-benzene-1,4-diamine ; N-1-(3-Diethylamino-propyl)-3-methyl-benzene-1,4-diamine ; N-1-(3-Dimethylamino-2,2-dimethyl-propyl)-3-methyl-benzene-1,4-diamine ; 2-Methyl-N-4-(2-piperidin-1-yl-ethyl)-benzene-1,4-diamine ; N-1-(2-Amino-ethyl)-2-methyl-benzene-1,4-diamine; N-4-(2-Amino-ethyl)-2-methyl-benzene-1,4-diamine.

11. Composé de formule (I) selon l'une des revendications précédentes, **caractérisé par le fait que** ce composé se présente sous la forme d'un sel.

12. Composé de formule (I) selon la revendication 11, **caractérisé par le fait qu'**il se présente sous la forme d'un sel d'addition avec un acide choisi parmi les chlorhydrates, les bromhydrates, les sulfates, les citrates, les succinates, les tartrates, les lactates, les tosylates, les benzènesulfonates, les phosphates et les acétates .

13. Composé nitré **caractérisé en ce qu'**il correspond à la formule (II) : 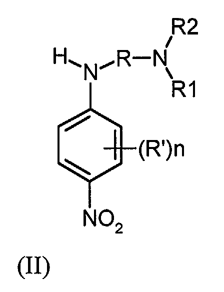 dans laquelle :
les radicaux R₁ et R₂ représentent, indépendamment l'un de l'autre, un atome d'hydrogène, un groupement alkyle, ou les radicaux R₁ et R₂ forment, ensemble et avec l'azote qu'ils substituent, un cycle saturé à 5 ou 6 chaînons éventuellement interrompu par un ou plusieurs atomes d'azote et/ou par un ou plusieurs groupements carbonyle, et éventuellement substitué par un ou plusieurs groupements alkyles,
le radical R représente un radical alkylène en C₂-C₁₀, linéaire ou ramifié, éventuellement interrompu par un ou plusieurs atomes choisis parmi l'azote ou l'oxygène, et éventuellement substitué par un ou plusieurs groupements choisis parmi les groupements amino, monoalkylamino, dialkylamino, alkylcarbonyle, amido, alkoxycarbonyle, monoalkylaminocarbonyle, dialkylaminocarbonyle,
le radical R' représente un radical choisi parmi les radicaux alkyle, alcoxy, alcoxyalkyle, monohydroxyalkyle , polyhydroxyalkyle et l'atome de chlore,
n est un entier égal à 1, sous réserve que ce composé ne soit pas le N-1(3-diethylaminopropyl)-3-methyl-4-nitro-1-aminobenzene et N-1-(3-pyrrolidino-propyl)3-methyl-4-nitro-1-aminobenzene.

14. Préparation du composé selon l'une des revendications 1 à 12 **caractérisé par le fait qu'**on effectue une étape de réduction d'un composé nitré correspondant.

15. Utilisation, pour la teinture des fibres, de préférence les fibres kératiniques telles que les cheveux, d'un composé de formule générale (I) selon l'une des revendications 1 à 12.

16. Composition cosmétique pour la teinture des fibres, de préférence les fibres kératiniques humaines telles que les cheveux, comprenant, dans un milieu approprié pour la teinture, au moins un composé de formule générale (I) selon l'une des revendications 1 à 12.

17. Composition selon la revendication 16 **caractérisée par le fait qu'**elle comprend au moins un adjuvant cosmétique choisi dans le groupe formé par les agents anti-oxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

18. Composition cosmétique pour la teinture des fibres, de préférence les fibres kératiniques telles que les cheveux, comprenant, dans un milieu approprié pour la teinture, au moins un composé de formule générale (I) selon l'une des revendications 1 à 12 et au moins un adjuvant cosmétique choisi dans le groupe formé par les agents antioxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

19. Composition selon l'une des revendications 16 à 18 **caractérisée par le fait que** la concentration du composé de formule générale (I) est comprise entre 0,0001 et 20%, de préférence entre 0,005 à 6% en poids par rapport au poids total de la composition.

20. Composition selon l'une des revendications 16 à 19 **caractérisée par le fait que** le milieu approprié pour la teinture est constitué par de l'eau ou par un mélange d'eau et d'au moins un solvant organique tel que, les alcools inférieurs en C₁-C₄, ramifiés ou non, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, le glycérol ainsi que les alcools aromatiques comme l'alcool benzylique ou le phénoxyéthanol, et leurs mélanges.

21. Composition selon l'une des revendications 17 à 20 **caractérisée par le fait que** la quantité en adjuvant cosmétique est comprise pour chacun d'eux entre 0,01 et 20 % en poids par rapport au poids de la composition.

22. Composition selon l'une des revendications 16 à 21 **caractérisée par le fait qu'**elle comprend au moins un précurseur de colorant d'oxydation additionnel différent des composés de formule (I).

23. Composition selon la revendication 22 **caractérisée par le fait que** le précurseur de colorant d'oxydation additionnel est un coupleur d'oxydation choisi parmi les méta-phénylènediamines, les méta-aminophénols, les méta-diphénols, les coupleurs naphtaléniques, les coupleurs hétérocycliques ainsi que leurs sels d'addition.

24. Composition selon la revendication 23 **caractérisée par le fait que** la concentration de ce coupleur est comprise entre 0,0001 et 20 % en poids par rapport au poids total de la composition.

25. Composition selon l'une des revendications 21 à 24 **caractérisée par le fait que** le précurseur de colorant d'oxydation additionnel est une base d'oxydation différente des composés de formule (I), choisie parmi les para-phénylènediamines, les bis-phénylalkylènediamines, les para-aminophénols, les ortho-aminophénols, les bases hétérocycliques et leurs sels d'addition.

26. Composition selon la revendication 25 **caractérisée par le fait que** la concentration de cette base d'oxydation est comprise entre 0,0001 et 20% en poids par rapport au poids total de la composition.

27. Composition selon l'une des revendications 16 à 26 **caractérisée par le fait qu'**elle renferme un ou plusieurs colorants directs naturels ou cationiques.

28. Composition prête à l'emploi, **caractérisée en ce qu'**il s'agit d'un mélange d'une composition selon l'une des revendications 16 à 27 avec au moins un agent oxydant choisi parmi le peroxyde d'hydrogène, le peroxyde d'urée, les bromates de métaux alcalins, les persels, les peracides et les enzymes oxydases.

29. Procédé de teinture des fibres kératiniques et en particulier des fibres kératiniques humaines telles que les cheveux, **caractérisée par le fait qu'**on applique sur lesdites fibres au moins une composition selon l'une des revendications 16 à 27, pendant une durée suffisante pour développer la coloration désirée en présence d'un agent oxydant, l'agent oxydant étant appliqué avant, simultanément ou après la composition.

30. Procédé de teinture des fibres kératiniques et en particulier des fibres kératiniques humaines telles que les cheveux, **caractérisée par le fait qu'**on applique sur lesdites fibres la composition prête à l'emploi selon la revendication 28, pendant une durée suffisante pour développer la coloration désirée.

31. Utilisation, pour la teinture des fibres, de préférence les fibres kératiniques telles que les cheveux, d'une composition comprenant, dans un milieu approprié pour la teinture, au moins un composé de formule générale (I) telle que défini à la revendication 1.

32. Utilisation de la composition selon la revendication 31 dans un dispositif à plusieurs compartiments, un premier compartiment contenant la composition cosmétique pour la teinture des fibres kératiniques et un deuxième compartiment contenant un agent oxydant
